(19) Europäisches Patentamt / European Patent Office / Office européen des brevets

(11) EP 1 613 146 B1

(12) # EUROPEAN PATENT SPECIFICATION

(45) Date of publication and mention of the grant of the patent: **19.06.2013 Bulletin 2013/25**

(21) Application number: **04741458.6**

(22) Date of filing: **14.04.2004**

(51) Int Cl.: *A01H 5/00* (2006.01) *C12N 15/82* (2006.01) *A01H 5/10* (2006.01) *C07K 14/825* (2006.01)

(86) International application number: **PCT/EP2004/050519**

(87) International publication number: **WO 2004/090142 (21.10.2004 Gazette 2004/43)**

(54) **PLANTS HAVING MODIFIED GROWTH CHARACTERISTICS AND METHOD FOR MAKING THE SAME**

PFLANZEN MIT MODIFIZIERTEN WACHSTUMSEIGENSCHAFTEN SOWIE HERSTELLUNGSVERFAHREN DAFÜR

PLANTES PRESENTANT DES CARACTERISTIQUES DE CROISSANCE MODIFIEES, ET LEUR PROCEDE DE PRODUCTION

(84) Designated Contracting States: **AT BE BG CH CY CZ DE DK EE ES FI FR GB GR HU IE IT LI LU MC NL PL PT RO SE SI SK TR**

(30) Priority: **14.04.2003 EP 03076086**

(43) Date of publication of application: **11.01.2006 Bulletin 2006/02**

(73) Proprietor: **CropDesign N.V.**
**9052 Zwijnaarde-Gent (BE)**

(72) Inventor: **SANZ MOLINERO, Ana, Isabel**
**B-9050 Gentbrugge (BE)**

(74) Representative: **Mistry, Meeta**
**Greaves Brewster LLP**
**Copa House**
**Station Road**
**Cheddar, BS27 3AH (GB)**

(56) References cited:
**WO-A-98/36084**

- **LUCCA PAOLA ET AL: "Approaches to improving the bioavailability and level of iron in rice seeds" JOURNAL OF THE SCIENCE OF FOOD AND AGRICULTURE, vol. 81, no. 9, July 2001 (2001-07), pages 828-834, XP001183265 ISSN: 0022-5142**
- **DATABASE EMBL 7 November 1991 (1991-11-07), TAKAHASHI: "A. thaliana AtMT-1 mRNA for metallothionein-like protein" XP002297021 Database accession no. X62818**
- **ZHOU J ET AL: "STRUCTURE, ORGANIZATION AND EXPRESSION OF THE METALLOTHIONEIN GENE FAMILY IN ARABIDOPSIS" MOL. GEN. GENET, XX, XX, vol. 248, 1995, pages 318-328, XP000907576**
- **PATER DE B S ET AL: "THE PROMOTER OF THE RICE GENE GOS2 IS ACTIVE IN VARIOUS DIFFERENT MONOCOT TISSUES AND BINDS RICE NUCLEAR FACTOR ASF-1" PLANT JOURNAL, BLACKWELL SCIENTIFIC PUBLICATIONS, OXFORD, GB, vol. 2, no. 6, 1992, pages 837-844, XP000907326 ISSN: 0960-7412 cited in the application**
- **COBBETT CHRISTOPHER ET AL: "Phytochelatins and metallothioneins: roles in heavy metal detoxification and homeostasis." ANNUAL REVIEW OF PLANT BIOLOGY. 2002, vol. 53, 2002, pages 159-182, XP002297019 ISSN: 1543-5008 cited in the application**
- **SUH M C ET AL: "Cadmium resistance in transgenic tobacco plants expressing the Nicotiana glutinosa L. metallothionein-like gene." MOLECULES AND CELLS. 31 DEC 1998, vol. 8, no. 6, 31 December 1998 (1998-12-31), pages 678-684, XP009036622 ISSN: 1016-8478**
- **KAERENLAMPI S ET AL: "Genetic engineering in the improvement of plants for phytoremediation of metal polluted soils" ENVIRONMENTAL POLLUTION, BARKING, GB, vol. 107, no. 2, 2000, pages 225-231, XP002287818 ISSN: 0269-7491**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- THOMAS JOHN C ET AL: "Yeast metallothionein in transgenic tobacco promotes copper uptake from contaminated soils." BIOTECHNOLOGY PROGRESS, vol. 19, no. 2, 21 November 2002 (2002-11-21), pages 273-280, XP002297020 ISSN: 8756-7938
- EVANS KATHERINE M ET AL: "Expression of the metallothionein-like gene PsMT-ALPHA in Escherichia coli and Arabidopsis thaliana and analysis of trace metal ion accumulation: Implications for PsMT-ALPHA function" PLANT MOLECULAR BIOLOGY, vol. 20, no. 6, 1992, pages 1019-1028, XP009036607 ISSN: 0167-4412
- FREISINGER EVA: "Plant MTs-long neglected members of the metallothionein superfamily.", 21 December 2008 (2008-12-21), DALTON TRANSACTIONS (CAMBRIDGE, ENGLAND : 2003) 21 DEC 2008, NR. 47, PAGE(S) 6663 - 6675 ISSN: 1477-9226

SEQUENCE LISTING

<110> CropDesign N.V.

<120> Plants having modified growth characteristics and method for making the same

<130> CD-089-PCT

<150> EP 03076086.2
<151> 2003-04-14

<160> 7

<170> PatentIn version 3.2

<210> 1
<211> 336
<212> DNA
<213> Arabidopsis thaliana

<220>
<221> misc_feature
<223> MT2a DNA sequence

<400> 1

```
ttttcattca taaatttttc ttcaatttga attttctcga gaaaaatgtc ttgctgtgga      60
ggaaactgcg gatgtggatc tggctgcaag tgcggcaacg gttgtggagg ttgcaaaatg     120
taccctgact tgggattctc cggcgagaca accacaactg agacttttgt cttgggcgtt     180
gcaccggcga tgaagaatca gtacgaggct tcaggggaga gtaacaacgc tgagaacgat     240
gcttgcaagt gtggatctga ctgcaagtgt gatccttgca cctgcaagtg aagaagcctt     300
tttaaataag cagagataat cgagtctctt taatta                     .         336
```

<210> 2
<211> 81
<212> PRT

<213> Arabidopsis thaliana

<220>
<221> MISC_FEATURE
<223> MT2a protein sequence

<400> 2

```
Met Ser Cys Cys Gly Gly Asn Cys Gly Cys Gly Ser Gly Cys Lys Cys
1               5                   10                  15

Gly Asn Gly Cys Gly Gly Cys Lys Met Tyr Pro Asp Leu Gly Phe Ser
            20                  25                  30

Gly Glu Thr Thr Thr Thr Glu Thr Phe Val Leu Gly Val Ala Pro Ala
        35                  40                  45

Met Lys Asn Gln Tyr Glu Ala Ser Gly Glu Ser Asn Asn Ala Glu Asn
    50                  55                  60

Asp Ala Cys Lys Cys Gly Ser Asp Cys Lys Cys Asp Pro Cys Thr Cys
65                  70                  75                  80

Lys
```

<210> 3
<211> 545
<212> DNA
<213> Arabidopsis thaliana

<220>
<221> misc_feature
<223> MT1 DNA sequence

<220>
<221> misc_feature
<222> (331)..(331)
<223> n is a, c, g, or t

<400> 3

```
cattcataaa tttttcttca atttgaattt tctcgagaaa aatgtcttgc tgtggaggaa     60
actgcggatg tggatctggc tgcaagtgcg gcaacggttg tggaggttgc aaaatgtacc    120
ctgacttggg attctccggc gagacaacca caactgagac ttttgtcttg ggcgttgcac    180
cggcgatgaa gaatcagtac gaggcttcag gggagagtaa caacgctgag agcgatgctt    240
gcaagtgtgg atctgactgc aagtgtgatc cttgcacctg caagtgaaga agcctttttta   300
aataagcaga gataatcgag tctctttaat ntaattaagt tattcaataa gtaaaccata    360
tataggatgg tgtttttagg tttggtttat gtgtaataat ggcttcagct tatcttttag    420
ccgatcattg tcttttgtgt ttgttttgat catatctttt agatgtctag caaatctgcc    480
atgtgatgag tttgtacttc cagtggaatg ataataatat tatagtttta aatcaaaaaa    540
aaaaa                                                                545
```

<210> 4
<211> 81
<212> PRT
<213> Arabidopsis thaliana

<220>
<221> MISC_FEATURE

<223> MT1 protein sequence

<400> 4

```
Met Ser Cys Cys Gly Gly Asn Cys Gly Cys Gly Ser Gly Cys Lys Cys
1               5                   10                  15

Gly Asn Gly Cys Gly Gly Cys Lys Met Tyr Pro Asp Leu Gly Phe Ser
            20                  25                  30

Gly Glu Thr Thr Thr Thr Glu Thr Phe Val Leu Gly Val Ala Pro Ala
        35                  40                  45

Met Lys Asn Gln Tyr Glu Ala Ser Gly Glu Ser Asn Asn Ala Glu Ser
    50                  55                  60

Asp Ala Cys Lys Cys Gly Ser Asp Cys Lys Cys Asp Pro Cys Thr Cys
65                  70                  75                  80

Lys
```

<210> 5
<211> 53
<212> DNA
<213> Artificial sequence

<220>
<223> primer prm03240

<400> 5
ggggacaagt ttgtacaaaa aagcaggctt cacaatgtct tgctgtggag gaa 53

<210> 6
<211> 47
<212> DNA
<213> Artificial sequence

<220>
<223> primer prm03241

<400> 6
ggggaccact ttgtacaaga aagctgggtt tcacttgcag gtgcaag 47

<210> 7
<211> 3032
<212> DNA
<213> Artificial sequence

<220>
<223> expression cassette for MT2a

<400> 7

```
aatccgaaaa gtttctgcac cgttttcacc ccctaactaa caatataggg aacgtgtgct     60
aaatataaaa tgagacctta tatatgtagc gctgataact agaactatgc aagaaaaact    120
catccaccta ctttagtggc aatcgggcta aataaaaaag agtcgctaca ctagtttcgt    180
tttccttagt aattaagtgg gaaaatgaaa tcattattgc ttagaatata cgttcacatc    240
tctgtcatga agttaaatta ttcgaggtag ccataattgt catcaaactc ttcttgaata    300
aaaaaatctt tctagctgaa ctcaatgggt aaagagagag atttttttta aaaaaataga    360
atgaagatat tctgaacgta ttggcaaaga tttaaacata taattatata attttatagt    420
ttgtgcattc gtcatatcgc acatcattaa ggacatgtct tactccatcc caatttttat    480
ttagtaatta aagacaattg acttattttt attatttatc ttttttcgat tagatgcaag    540
gtacttacgc acacactttg tgctcatgtg catgtgtgag tgcacctcct caatacacgt    600
tcaactagca acacatctct aatatcactc gcctatttaa tacatttagg tagcaatatc    660
tgaattcaag cactccacca tcaccagacc acttttaata atatctaaaa tacaaaaaat    720
aattttacag aatagcatga aaagtatgaa acgaactatt taggtttttc acatacaaaa    780
aaaaaaagaa ttttgctcgt gcgcgagcgc caatctccca tattgggcac acaggcaaca    840
acagagtggc tgcccacaga acaacccaca aaaaacgatg atctaacgga ggacagcaag    900
tccgcaacaa ccttttaaca gcaggctttg cggccaggag agaggaggag aggcaaagaa    960
aaccaagcat cctcctcctc ccatctataa attcctcccc ccttttcccc tctctatata   1020
ggaggcatcc aagccaagaa gagggagagc accaaggaca cgcgactagc agaagccgag   1080
cgaccgcctt cttcgatcca tatcttccgg tcgagttctt ggtcgatctc ttccctcctc   1140
cacctcctcc tcacagggta tgtgcccttc ggttgttctt ggatttattg ttctaggttg   1200
tgtagtacgg gcgttgatgt taggaaaggg gatctgtatc tgtgatgatt cctgttcttg   1260
gatttgggat agaggggttc ttgatgttgc atgttatcgg ttcggtttga ttagtagtat   1320
ggttttcaat cgtctggaga gctctatgga aatgaaatgg tttagggtac ggaatcttgc   1380
gattttgtga gtaccttttg tttgaggtaa aatcagagca ccggtgattt tgcttggtgt   1440
aataaaagta cggttgtttg gtcctcgatt ctggtagtga tgcttctcga tttgacgaag   1500
ctatcctttg tttattccct attgaacaaa aataatccaa ctttgaagac ggtcccgttg   1560
atgagattga atgattgatt cttaagcctg tccaaaattt cgcagctggc ttgtttagat   1620
acagtagtcc ccatcacgaa attcatggaa acagttataa tcctcaggaa caggggattc   1680
cctgttcttc cgatttgctt tagtcccaga atttttttc ccaaatatct taaaaagtca   1740
ctttctggtt cagttcaatg aattgattgc tacaaataat gcttttatag cgttatccta   1800

gctgtagttc agttaatagg taatacccct atagtttagt caggagaaga acttatccga   1860
tttctgatct ccatttttaa ttatatgaaa tgaactgtag cataagcagt attcatttgg   1920
attatttttt ttattagctc tcaccccttc attattctga gctgaaagtc tggcatgaac   1980
tgtcctcaat tttgttttca aattcacatc gattatctat gcattatcct cttgtatcta   2040
cctgtagaag tttctttttg gttattcctt gactgcttga ttacagaaag aaatttatga   2100
agctgtaatc gggatagtta tactgcttgt tcttatgatt catttccttt gtgcagttct   2160
tggtgtagct tgccactttc accagcaaag ttcatttaaa tcaactaggg atatcacaag   2220
tttgtacaaa aaagcaggct tcacaatgtc ttgctgtgga ggaaactgcg gatgtggatc   2280
tggctgcaag tgcggcaacg gttgtggagg ttgcaaaatg taccctgact tgggattctc   2340
cggcgagaca accacaactg agacttttgt cttgggcgtt gcaccggcga tgaagaatca   2400
gtacgaggct tcaggggaga gtaacaacgc tgagaacgat gcttgcaagt gtggatctga   2460
ctgcaagtgt gatccttgca cctgcaagtg aaacccagct ttcttgtaca aagtggtgat   2520
atcacaagcc cgggcggtct tctagggata acagggtaat tatatccctc tagatcacaa   2580
gcccgggcgg tcttctacga tgattgagta ataatgtgtc acgcatcacc atgggtggca   2640
gtgtcagtgt gagcaatgac ctgaatgaac aattgaaatg aaaagaaaaa aagtactcca   2700
tctgttccaa attaaaattc attttaacct tttaataggt ttatacaata attgatatat   2760
gttttctgta tatgtctaat ttgttatcat ccgggcggtc ttctagggat aacagggtaa   2820
ttatatccct ctagacaaca cacaacaaat aagagaaaaa acaaataata ttaatttgag   2880
aatgaacaaa aggaccatat cattcattaa ctcttctcca tccatttcca tttcacagtt   2940
cgatagcgaa aaccgaataa aaaacacagt aaattacaag cacaacaaat ggtacaagaa   3000
aaacagtttt cccaatgcca taatactcga ac                                 3032
```

## Description

### Field of the invention

The present invention concerns a method for increasing seed yield in plants. More specifically, the present invention concerns a method for increasing seed yield in plants, comprising introduction and overexpression in a plant of a nucleic acid encoding a metallothionein protein as described herein. The present invention also concerns plants having increased expression of a nucleic acid sequence encoding a metallothionein, which plants have increased seed yield relative to corresponding wild type plants. Also described herein are constructs suitable for use in the methods of the invention.

### Background of the invention

Some heavy metals, particularly copper and zinc, are essential micronutrients that play a role in a range of plant physiological processes via the action of Cu- and Zn-dependent enzymes. These and other nonessential heavy metal ions, such as cadmium, lead, and mercury, are highly reactive and consequently can be toxic to living cells. Thus plants, like all living organisms, have evolved a suite of mechanisms that control and respond to the uptake and accumulation of both essential and nonessential heavy metals. These mechanisms include the chelation and sequestration of heavy metals by particular ligands. The two best-characterized heavy metal-binding ligands in plant cells are the phytochelatins (PCs) and metallothioneins (MTs). MTs are cysteine-rich polypeptides encoded by a family of genes. In contrast, PCs are a family of enzymatically synthesized cysteine-rich peptides.

Metallothioneins, products of mRNA translation, are low molecular weight, cysteine-rich, metal-binding proteins. MT proteins and genes are found throughout the animal and plant kingdoms as well as in the prokaryote *Synechococcus.* The large number of cysteine residues in MTs bind a variety of metals by mercaptide bonds. MTs typically contain two metal-binding, cysteine-rich domains that give these metalloproteins a dumbbell conformation. The data available today tends to support a role for MTs in copper tolerance (as PCs protect against cadmium).

The classification of MT proteins is based on the arrangement of Cys residues. Cobbett and Goldsbrough (Annu Rev Plant Biol. 53, 159-82, 2002) discriminate four classes: Type 1 to Type 4. Type 2 MTs contain two cysteine rich domains separated by a spacer of approximately 40 amino acids, with the first pair of cysteines present as a Cys-Cys motif in amino acid positions 3 and 4. In addition, the sequences of the N-terminal domain of Type 2 MTs (MSCCGGNCGCS-) are highly conserved.

*Arabidopsis,* rice and sugarcane contain genes encoding all four types of MTs. General observations may be made about the expression pattern of these genes. Type 4 MTs are restricted to developing seeds. Type 1 MT expression tends to be higher in roots than shoots, whereas generally the reverse is observed for Type 2 MTs. Type 3 MTs are expressed in fleshy fruits or in leaves of non-fleshy fruit producing plants (like *Arabidopsis*). Various environmental factors influence the expression of these genes.

Transgenics plants expressing GUS under the control of the AtMT2a promoter have been produced. In young plants, staining was found only in cotyledons and lateral root tips. GUS activity in older plants was seen at the base of trichomes, in hydathodes, sepals, anthers, stigma, root tips, and vascular tissues at branch points of lateral roots. As leaves aged, GUS staining increased.

Transgenic plants expressing MTs have been produced serving two purposes:

1) Phytoremediation of heavy metal contaminated soils: transgenic tobacco and *Brassica* plants, engineered to overexpress MTs, moderately resist the toxicity of heavy metals like cadmium (Suh et al., Mol Cells. 8, 678-684, 1998).
2) Nutritional quality improvement, when co-expressed in rice with phytase and ferritin, to improve iron diet in humans. The metallothionein-like protein, rich in cysteine, (a sulfur-rich amino acid) helps in iron absorption by the human digestive system (Potrykus I (2002) Nutritional improvement of rice to reduce malnutrition in developing countries. In "Plant Biotechnology 2002 and Beyond" I.K. Vasil (ed.) pp 401-406).

Published PCT patent application number WO 98/36084 in the name of Agricola Technologies Inc. describes enhancing plant growth using genes encoding metal-binding proteins, such as metallothionein. However, closer inspection of the patent application seems to suggest that the metallothionein-encoding genes would need to be expressed in combination with other genes in order for the enhanced plant growth to be realised.

Given the ever-increasing world population, it remains a major goal of agricultural research to improve the efficiency of agriculture and to increase the diversity of plants in horticulture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic complements that may not always result in the desirable trait being passed on from parent plants. Advances in molecular biology have allowed mankind to manipulate the germplasm of animals and plants. Genetic engineering of plants entails the isolation and manipulation of genetic material (typically in the form of DNA or RNA) and the subsequent introduction of that genetic material into a plant. Such technology has led to the development of plants having various improved economic, agronomic or horticultural traits. A trait of particular economic interest is high yield.

**Detailed description**

It has now surprisingly been found that overexpression in a plant of a nucleic acid encoding a metallothionein protein as described herein gives rise to plants having increased seed yield. Therefore according to the present invention there is provided a method for increasing seed yield, comprising introducing and overexpressing in a plant an isolated nucleic acid encoding a metallothionein protein as described herein.

Also described is the introduction of a genetic modification in a plant and selecting for modulated expression in a plant of a nucleic acid encoding a metallothionein protein, provided that the modified growth and development is not increased metal accumulation or increased tolerance or resistance to abiotic stress. With increased metal accumulation is meant any uptake of metals for the purpose of bioremediation or for improving the nutritional quality of plants. In particular, the uptake of heavy metals or iron is envisaged. The term abiotic stress stands for stresses caused by salt, cold or osmotic pressure and includes also oxidative stress.

Described herein are methods encompassing a genetic modification of a plant or a plant cell. The term "genetic modification" refers to a change by human intervention in the genetic content of a cell compared to a wild type cell and includes techniques like genetic engineering, breeding or mutagenesis. The change in genetic content comprises modifications of the genome and includes addition, deletion and substitution of genetic material in the chromosomes of a plant cell as well as in episomes. The term also encompasses the addition of extrachromosomal information to a plant cell. Preferably, the genetic modification results in modulated expression of a nucleic acid. The methods also encompass a subsequent step of selection, during which plants with the desired characteristics are selected. The selection step may be based on monitoring the presence or absence of modified growth characteristics, or on monitoring the presence or absence of selectable or screenable marker genes linked an introduced nucleic acid of interest.

In the present invention increased expression, of a nucleic acid is envisaged. Increasing expression of a nucleic acid encoding a metallothionein or increasing the activity and/or levels of the metallothionein itself encompasses increased expression of a gene and/or increased activity and/or levels of a gene product, namely a polypeptide, in specific cells or tissues. Altered expression of a gene and/or altered activity and/or levels of a gene product is effected, by recombinant means. Modulating expression of a gene and/or levels of a gene product and/or modulating activity of a gene product may be effected directly through the modulation of expression of a metallothionein-encoding gene and/or directly through the modulation of the activity and/or levels of a metallothionein protein. The modulated expression may result from altered expression of a metallothionein encoding nucleic acid that was previously introduced into a plant. Similarly, modulated levels and/or activity of a metallothionein protein may result from altered expression of a metallothionein encoding nucleic acid that was previously introduced into a plant. Additionally, the modulation of expression as mentioned above is effected in an indirect way, for example may be effected as a result of decreased or increased levels and or activity of factors that control the expression of a metallothionein gene or that influence the activity and/or levels of the metallothionein.

Advantageously, modulation of expression of a nucleic acid encoding a metallothionein and/or modulation of activity and/or levels of the metallothionein itself may be effected by chemical means, i.e. by exogenous application of one or more compounds or elements capable of modulating activity and/or levels of the metallothionein protein and/or capable of modulating expression of a metallothionein encoding nucleic acid (which nucleic acid is a transgene introduced into a plant). The term "exogenous application" taken in its broadest context includes contacting or administering cells, tissues, organs or organisms with a suitable compound or element. The compound may be applied to a plant in a suitable form for uptake (such as through application to the soil for uptake via the roots, or by applying directly to the leaves, for example by spraying).

Suitable compounds or elements for exogenous application include metallothionein encoding nucleic acids and nucleic acids that hybridise therewith; the metallothionein gene product or a homologue, derivative or active fragment thereof and/or antibodies recognizing or mimicking the gene product. Such antibodies may comprise "plantibodies", single chain antibodies, IgG antibodies and heavy chain antibodies from camels or other members of the Camelidae, as well as fragments thereof. Additionally or alternatively, contacting or administering cells, tissues, organs or organisms with an interacting protein or with an inhibitor or activator of the gene/gene product provides another exogenous means for modulation of expression of a nucleic acid encoding a metallothionein and/or for modulation of activity and/or level of the metallothionein itself. Modulation of expression of a nucleic acid encoding a metallothionein protein and/or modulation of activity and/or levels of the metallothionein itself may also be effected as a result of altered levels of factors that directly or indirectly activate or inactivate a metallothionein protein.

Plants, seeds or other plant material can also be subjected to treatment with mutagenic substances. Chemical substances effecting mutagenesis comprise N-nitroso-N-ethylurea, ethylene imine, ethyl methanesulphonate or diethyl sulphate. As an alternative, ionising radiation such as y-rays or X-rays can equally well be used. Methods for introducing mutations and testing the effect of mutations (such as modified protein expression and/or modified protein activity) are known in the art. Encompassed by mutagenesis are methods employing chemical mutagens, as well as physical mutagens, such as radiation.

Any characteristic of the metallothionein can be altered by mutagenesis. For example, the expression level can be increased or decreased, the activity of the protein can be modified, or the metal binding properties can be adapted.

Also described herein is modulation of expression of a nucleic acid sequence encoding a metallothionein and/or modulation of activity of the metallothionein itself effected by recombinant means. Such recombinant means may comprise a direct and/or indirect approach for modulation of expression of a nucleic acid sequence and/or for modulation of the activity of a protein.

For example, an indirect approach may comprise introducing into a plant, a nucleic acid sequence capable of modulating activity and/or levels of the protein in question (a metallothionein) and/or expression of the gene in question (a gene encoding a metallothionein). Examples of such nucleic acids to be introduced into a plant are nucleic acids encoding transcription factors, activators or inhibitors that bind to the promoter of the metallothionein gene or that interact with the metallothionein protein. Methods to test these kinds of interaction and to isolate the nucleic acids encoding these interactors are for example yeast one-hybrid or yeast two-hybrid screening. Also encompassed by an indirect approach for modulating activity of a metallothionein and/or expression of a metallothionein gene is the inhibition or stimulation of regulatory sequences that drive expression of the native gene or transgene. Such regulatory sequences may be introduced into a plant. Furthermore, modulation of the activity of a metallothionein may be effected by altering levels in a plant of a factor able to interact with metallothionein. Such factors may include ligands (regulators, subunits, substrates, targets) of the metallothionein.

According to the present invention, increasing expression of a metallothionein gene or increasing the activity and/or levels of a metallothionein protein comprises introducing and overexpressing in a plant a nucleic acid sequence encoding a metallothionein polypeptide as defined herein. The nucleic acid sequence may be introduced into a plant by, for example, transformation.

According to the present invention expression of a nucleic acid is enhanced or increased. Methods for obtaining enhanced or increased expression of genes or gene products are well documented in the art and include, for example, overexpression driven by a (strong) promoter, the use of transcription enhancers or translation enhancers. The term overexpression as used herein means any form of expression that is additional to the original wild-type expression level. Preferably the nucleic acid to be introduced into the plant and/or the nucleic acid that is to be overexpressed in the plants is in a sense direction with respect to the promoter to which it is operably linked. The nucleic acid to be overexpressed encodes a type 2 metallothionein as defined below, further preferably the nucleic acid sequence encoding the metallothionein is isolated from a plant, preferably from a dicotyledonous plant, preferably of the family Brassicaceae, further preferably the sequence is isolated from *Arabidopsis thaliana,* most preferably the nucleic acid sequence is as represented by SEQ ID NO: 1 or a portion thereof, or encodes an amino acid sequence as represented by SEQ ID NO: 2 or a homologue, derivative or active fragment thereof. It should be noted that the applicability of the invention does not rest upon the use of the nucleic acid represented by SEQ ID NO: 1, nor upon the nucleic acid sequence encoding the amino acid sequence of SEQ ID NO: 2, but that other nucleic acid sequences encoding homologues, derivatives or active fragments of SEQ ID NO: 2, or portions of SEQ ID NO: 1, or sequences hybridising with SEQ ID NO: 1 may be used in the methods of the present invention. In particular, homologues from other plant species such as tobacco, maize or rice are also useful in the present invention.

Also described herein is increased expression of a metallothionein encoding gene or increased activities and/or levels of a metallothionein protein in a plant cells achieved by mutagenesis of the plant cell. For example these mutations can be responsible for the altered control of a metallothionein-encoding gene, resulting in higher expression of the gene. Mutations can also cause conformational changes of the protein, resulting in higher activity and/or levels of the protein.

The term metallothionein includes proteins homologous to the metallothionein as presented in SEQ ID NO 2. Metallothioneins are well known in the art, for a recent overview and classification, see Cobbett and Goldsbrough (2002). Metallothioneins are small proteins with a dumbbell conformation that finds its origin in conserved N-terminal and C-terminal cysteine rich domains which are separated from each other by a region that is variable in length and amino acid composition. Based on the primary structure 4 types of metallothioneins are discriminated, an alignment of various plant metallothioneins is given in Figure 1. The metallothionein of SEQ ID NO 2 comprises a conserved N-terminal domain typical for type 2 metallothioneins as defined by Cobbett and Goldsbrough (2002), which domain comprises the consensus sequence "MSCCGG (N/S) CGCG (T/S/A) (G/A/S) C (K/Q/S) C", accordingly, homologues to be used in the methods of the present invention are metallothioneins comprising this conserved domain. Additionally, the metallothionein homologues have metal binding activity which can be measured in a metal saturation test (Scheuhammer et al., Toxicol. Appl Pharmacol. 82, 417-425, 1986) and/or may function as a redox sensor (Fabisiak et al., Methods Enzymol. 353, 268-281 (2002)).

Methods for the search and identification of metallothionein homologues would be well within the realm of a person skilled in the art. Such methods comprise comparison of the sequences represented by SEQ ID NO 1 or 2, in a computer readable format, with sequences that are available in public databases such as MIPS (http://mips.gsf.de/), GenBank (http://www.ncbi.nlm.nih.gov/Genbank/index.html) or EMBL Nucleotide Sequence Database (http://www.ebi.ac.uk/embl/index.html), using algorithms well known in the art for the alignment or comparison of sequences, such as GAP (Needleman and Wunsch, J. Mol. Biol. 48; 443-453 (1970)), BESTFIT (using the local homology algorithm of Smith and Waterman (Advances in Applied Mathematics 2; 482-489 (1981))), BLAST (Altschul, S.F., Gish, W., Miller, W., Myers, E.W. & Lipman, D.J., J. Mol. Biol. 215:403-410 (1990)), FASTA and TFASTA (W. R. Pearson and D. J. Lipman Proc.Natl.Acad.Sci. USA 85:2444- 2448 (1988)). The software for performing BLAST analysis is publicly available through the National Centre for Biotechnology Information. The abovementioned homologues were identified using blast default parameters (BLOSUM62 matrix, gap opening penalty 11 and gap extension penalty 1) and preferably the full length sequences are used for analysis.

"Homologues" of a metallothionein protein encompass peptides, oligopeptides, polypeptides, proteins and enzymes having amino acid substitutions, deletions and/or insertions relative to the unmodified protein in question and having similar biological and functional activity as the unmodified protein from which they are derived. To produce such homologues, amino acids of the protein may be replaced by other amino acids having similar properties (such as similar hydrophobicity, hydrophilicity, antigenicity, propensity to form or break $\alpha$-helical structures or $\beta$-sheet structures). Conservative substitution tables are well known in the art (see for example Creighton (1984) Proteins. W.H. Freeman and Company). The homologues useful in the method according to the invention have at least 50% sequence identity or similarity (functional identity) to the unmodified protein, alternatively at least 60% sequence identity or similarity to an unmodified protein, alternatively at least 70% sequence identity or similarity to an unmodified protein. Typically, the homologues have at least 80% sequence identity or similarity to an unmodified protein, preferably at least 85% sequence identity or similarity, further preferably at least 90% sequence identity or similarity to an unmodified protein, most preferably at least 95% sequence identity or similarity to an unmodified protein. Metallothionein homologues include the proteins comprising the conserved sequence "MSCCGG (N/S) CGCG (T/S/A) (G/A/S) C (K/Q/S) c", such as SEQ ID NO 4 or GenBank accessions CAA71803, AAP94016, CAA71804, NP_195858, AAM62956, AAB61212, CAA65009, CAA92243.

Homologous proteins can be grouped in "protein families". A protein family can be defined by functional and sequence similarity analysis, such as, for example, Clustal W. A neighbour-joining tree of the proteins homologous to metallothionein can be generated by the Clustal W program and gives a good overview of its structural and ancestral relationship. In a particular embodiment of the present invention, the metallothionein homologues belong to the type 2 metallothionein protein family (Cobbett and Goldsbrough, 2002). In the *Arabidopsis* genome two type 2 family members of the metallothionein protein were identified (GenBank accessions AAA50250, NP_195858). Also in other plants such as rice or other monocotyledonous plants, family members of the metallothionein may be identified. Advantageously also these family members are useful in the methods of the present invention.

Two special forms of homology, orthologous and paralogous, are evolutionary concepts used to describe ancestral relationships of genes. The term "paralogous" relates to homologous genes that result from one or more gene duplications within the genome of a species. The term "orthologous" relates to homologous genes in different organisms due to ancestral relationship of these genes. The term "homologues" as used herein also encompasses paralogues and orthologues of the proteins useful in the methods according to the invention.

Orthologous genes can be identified by querying one or more gene databases with a query gene of interest, using for example the BLAST program. The highest-ranking subject genes that result from the search are then again subjected to a BLAST analysis, and only those subject genes that match again with the query gene are retained as true orthologous genes. For example, to find a rice orthologue of an *Arabidopsis thaliana* gene, one may perform a BLASTN or TBLASTX analysis on a rice database (such as (but not limited to) the *Oryza sativa* Nipponbare database available at the NCBI (http://www.ncbi.nlm.nih.gov) or the genomic sequences of rice (cultivars indica or japonica)). In a next step, the obtained rice sequences are used in a reverse BLAST analysis using an *Arabidopsis* database. The results may be further refined when the resulting sequences are analysed with ClustalW and visualised in a neighbour joining tree. The method can be used to identify orthologues from many different species.

"Homologues" of a metallothionein encompass proteins having amino acid substitutions, insertions and/or deletions relative to the unmodified protein. "Substitutional variants" of a protein are those in which at least one residue in an amino acid sequence has been removed and a different residue inserted in its place. Amino acid substitutions are typically of single residues, but may be clustered depending upon functional constraints placed upon the polypeptide; insertions will usually be of the order of about 1 to 10 amino acid residues, and deletions will range from about 1 to 20 residues. Preferably, amino acid substitutions comprise conservative amino acid substitutions. "Insertional variants" of a protein are those in which one or more amino acid residues are introduced into a predetermined site in a protein. Insertions can comprise amino-terminal and/or carboxy-terminal fusions as well as intra-sequence insertions of single or multiple amino acids. Generally, insertions within the amino acid sequence will be smaller than amino- or carboxy-terminal fusions, of the order of about 1 to 10 residues. Examples of amino- or carboxy-terminal fusion proteins or peptides include the binding domain or activation domain of a transcriptional activator as used in the yeast two-hybrid system, phage coat proteins, (histidine)6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope. "Deletion variants" of a protein are characterised by the removal of one or more amino acids from the protein. Amino acid variants of a protein may readily be made using peptide synthetic techniques well known in the art, such as solid phase peptide synthesis and the like, or by recombinant DNA manipulations. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

The term "derivatives" refers to peptides, oligopeptides, polypeptides, proteins and enzymes which may comprise substitutions, deletions or additions of naturally and non-naturally occurring amino acid residues compared to the amino acid sequence of a naturally-occurring form of the protein, for example, as presented in SEQ ID NO: 2. "Derivatives" of a metallothionein protein encompass peptides, oligopeptides, polypeptides, proteins and enzymes which may comprise naturally occurring altered, glycosylated, acylated or non-naturally occurring amino acid residues compared to the amino acid sequence of a naturally-occurring form of the polypeptide. A derivative may also comprise one or more non-amino acid substituents compared to the amino acid sequence from which it is derived, for example a reporter molecule or other ligand, covalently or non-covalently bound to the amino acid sequence such as, for example, a reporter molecule which is bound to facilitate its detection, and non-naturally occurring amino acid residues relative to the amino acid sequence of a naturally-occurring protein.

"Active fragments" or "functional fragments" of a metallothionein encompass at least 15, preferably 20, 25, more preferably 30 or more contiguous amino acid residues of a protein, which residues retain similar biological and/or functional activity to the naturally occurring protein. A fragment of a metallothionein protein comprises at least the conserved N-terminal domain specified above.

The term metallothionein encoding nucleic acid/gene, as defined herein, refers to any nucleic acid or the complement thereof encoding a metallothionein protein comprising the consensus sequence MSCCGG (N/S) CGCG (T/S/A) (G/A/S) C (K/Q/S) C. The nucleic acid may be derived (either directly or indirectly (if subsequently modified)) from any source provided that the nucleic acid, when expressed in a plant, leads to over expression of a metallothionein nucleic acid/gene or increased activity and/or levels of a metallothionein. The nucleic acid may be isolated from an eukaryotic source, such as plants (including algae). This nucleic acid may be substantially modified from its native form in composition and/or genomic environment through deliberate human manipulation. The nucleic acid sequence is preferably a homologous nucleic acid sequence, i.e. a structurally and/or functionally related nucleic acid sequence, preferably obtained from a plant, whether from the same plant species or different. The nucleic acid sequence may be isolated from a dicotyledonous plant species, preferably from the family Brassicaceae, further preferably from *Arabidopsis thaliana.* More preferably, the nucleic acid is as represented by SEQ ID NO: 1 or a portion thereof or a nucleic acid sequence capable of hybridising therewith, which hybridising sequence encodes proteins having metallothionein protein activity, i.e. similar biological activity to that of SEQ ID NO: 1, or it is a nucleic acid encoding an amino acid represented by SEQ ID NO: 2 or encoding a homologue, derivative or active fragment thereof. This term also encompasses variants of the nucleic acid encoding a metallothionein protein due to the degeneracy of the genetic code; allelic variants of the nucleic acid encoding a metallothionein; and different splice variants of the nucleic acid encoding a metallothionein and variants that are interrupted by one or more intervening sequences.

Advantageously, the method according to the present invention may also be practised using portions of a DNA or nucleic acid sequence, which portions encode a peptide that retains metallothionein activity, i.e. a similar biological function to that of SEQ ID NO: 2. Portions of a DNA sequence refer to a piece of DNA derived or prepared from an original (larger) DNA molecule, which DNA portion, when expressed in a plant, gives rise to plants having increased seed yield. The portion may comprise many genes, with or without additional control elements, or may contain just spacer sequences etc.

The present invention also encompasses nucleic acid sequences capable of hybridising with a nucleic acid sequence encoding a metallothionein, which nucleic acid sequences may also be useful in practising the methods according to the invention. The term "hybridisation" as defined herein is a process wherein substantially homologous complementary nucleotide sequences anneal to each other. The hybridisation process can occur entirely in solution, i.e. both complementary nucleic acids are in solution. Tools in molecular biology relying on such a process include the polymerase chain reaction (PCR; and all methods based thereon), subtractive hybridisation, random primer extension, nuclease S1 mapping, primer extension, reverse transcription, cDNA synthesis, differential display of RNAs, and DNA sequence determination. The hybridisation process can also occur with one of the complementary nucleic acids immobilised to a matrix such as magnetic beads, Sepharose beads or any other resin. Tools in molecular biology relying on such a process include the isolation of poly ($A^+$) mRNA. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). Tools in molecular biology relying on such a process include RNA and DNA gel blot analysis, colony hybridisation, plaque hybridisation, *in situ* hybridisation and micro array hybridisation. In order to allow hybridisation to occur, the nucleic acid molecules are generally thermally or chemically denatured to melt a double strand into two single strands and/or to remove hairpins or other secondary structures from single stranded nucleic acids. The stringency of hybridisation is influenced by conditions such as temperature, salt concentration and hybridisation buffer composition.
For applications requiring high selectivity, one will typically desire to employ relatively stringent conditions to form the hybrids, e.g., one will select relatively low salt and/or high temperature conditions, such as provided by about 0.02 M to about 0.15 M NaCl at temperatures of about 50°C to about 70°C.
High stringency conditions for hybridisation thus include high temperature and/or low salt concentration (salts include NaCl and $Na_3$-citrate) but can also be influenced by the inclusion of formamide in the hybridisation buffer and/or lowering the concentration of compounds such as SDS (sodium dodecyl sulphate) in the hybridisation buffer and/or exclusion of compounds such as dextran sulphate or polyethylene glycol (promoting molecular crowding) from the hybridisation buffer. Sufficiently low stringency hybridisation conditions are particularly preferred for the isolation of nucleic acids homologous to the DNA sequences of the invention defined supra. Elements contributing to homology include allelism, degeneration of the genetic code and differences in preferred codon usage.

"Stringent hybridisation conditions" and "stringent hybridisation wash conditions" in the context of nucleic acid hybridisation experiments such as Southern and Northern hybridisations are sequence dependent and are different under different environmental parameters. For example, longer sequences hybridise specifically at higher temperatures. The $T_m$ is the temperature under defined ionic strength and pH, at which 50% of the target sequence hybridises to a perfectly matched probe. Specificity is typically the function of post-hybridisation washes. Critical factors of such washes include the ionic strength and temperature of the final wash solution.

Generally, stringent conditions are selected to be about 50°C lower than the thermal melting point ($T_m$) for the specific sequence at a defined ionic strength and pH. The $T_m$ is the temperature under defined ionic strength and pH, at which 50% of the target sequence hybridises to a perfectly matched probe. The $T_m$ is dependent upon the solution conditions and the base composition of the probe, and may be calculated using the following equation:

$$T_m = 79.8°C + (18.5x\log[Na^+]) + (58.4°Cx\%[G+C]) - (820x(\#bp\ in\ duplex)^{-1}) - (0.5x\%\ formamide)$$

More preferred stringent conditions are when the temperature is 20°C below $T_m$, and the most preferred stringent conditions are when the temperature is 10°C below $T_m$. Non-specific binding may also be controlled using any one of a number of known techniques such as, for example, blocking the membrane with protein containing solutions, additions of heterologous RNA, DNA, and SDS to the hybridisation buffer, and treatment with Rnase.

Wash conditions are typically performed at or below hybridisation stringency. Generally, suitable stringent conditions for nucleic acid hybridisation assays or gene amplification detection procedures are as set forth above. More or less stringent conditions may also be selected.

For the purposes of defining the level of stringency, reference can conveniently be made to Sambrook et al. (2001) Molecular Cloning: a laboratory manual, 3rd Edition Cold Spring Harbor Laboratory Press, CSH, New York or to Current Protocols in Molecular Biology, John Wiley & Sons, N.Y. (1989). An example of low stringency conditions is 4-6x SSC / 0.1-0.5% w/v SDS at 37-45°C for 2-3 hours. Depending on the source and concentration of the nucleic acid involved in the hybridisation, alternative conditions of stringency may be employed such as medium stringent conditions. Examples of medium stringent conditions include 1-4x SSC / 0.25% w/v SDS at ≥ 45°C for 2-3 hours. An example of high stringency conditions includes 0.1-1x SSC /0.1% w/v SDS at 60°C for 1-3 hours. The skilled artisan is aware of various parameters which may be altered during hybridisation and washing and which will either maintain or change the stringency conditions. For example, another stringent hybridisation condition is hybridisation at 4x SSC at 65°C, followed by a washing in 0.1 x SSC, at 65°C for about one hour. Alternatively, an exemplary stringent hybridisation condition is in 50% formamide, 4x SSC at 42°C. Still another example of stringent conditions include hybridisation at 62°C in 6x SSC, 0.05x BLOTTO and washing at 2x SSC, 0.1% w/v SDS at 62°C.

Described herein are methods practised using an alternative splice variant of a nucleic acid sequence encoding a metallothionein. The term "alternative splice variant" as used herein encompasses variants of a nucleic acid sequence in which selected introns and/or exons have been excised, replaced or added. Such variants will be ones in which the biological activity of the protein remains unaffected, which can be achieved by selectively retaining functional segments of the protein. Such splice variants may be found in nature or can be manmade. Methods for making such splice variants are well known in the art. Therefore described herein a method for modifying the growth characteristics of plants, comprising modulating expression in a plant of an alternative splice variant of a nucleic acid sequence encoding a metallothionein protein and/or by modulating activity and/or levels of a metallothionein protein encoded by the alternative splice variant. Preferably, the splice variant is a splice variant of the sequence represented by SEQ ID NO: 1.

Described herein are methods practised using allelic variants of a nucleic acid sequence encoding a metallothionein, preferably an allelic variant of a sequence represented by SEQ ID NO: 1. Allelic variants exist in nature and encompassed within the methods of the present invention is the use of these natural alleles. Allelic variants encompass Single Nucleotide Polymorphisms (SNPs), as well as Small Insertion/Deletion Polymorphisms (INDELs). The size of INDELs is usually less than 100 bp). SNPs and INDELs form the largest set of sequence variants in naturally occurring polymorphic strains of most organisms. They are helpful in mapping genes and discovery of genes and gene functions. They are furthermore helpful in identification of genetic loci, e.g. plant genes, involved in determining processes such as growth rate, plant size and plant yield, plant vigor, disease resistance, stress tolerance etc. Many techniques are nowadays available to identify SNPs and/or INDELs including (i) PCR followed by denaturing high performance liquid chromatography (DHPLC; e.g. Cho et al. (1999) Nature Genet. 23, 203-207); (ii) constant denaturant capillary electrophoresis (CDCE) combined with high-fidelity PCR (e.g. Li-Sucholeiki et al. (1999) Electrophoresis 20, 1224-1232); (iii) denaturing gradient gel electrophoresis (Fischer and Lerman (1983) Proc. Natl. Acad. Sci. USA 80, 1579-1583); (iv) matrix-assisted laser desorption/ionization time-of-flight mass spectrometry (MALDI-TOF MS; e.g. Ross et al. (2000) Biotechniques 29, 620-629); (v) real-time fluorescence monitoring PCR assays (Tapp et al. (2000) Biotechniques 28, 732-738); (vi) AcryditeTM gel technology (Kenney et al. (1998) Biotechniques 25, 516-521); (vii) cycle dideoxy fingerprinting (CddF; Langemeier et al. (1994) Biotechniques 17, 484-490); (viii) single-strand conformation polymorphism (SSCP) analysis (Vidal-Puig and Moller (1994) Biotechniques 17, 490-496) and (ix) mini-sequencing primer extension reaction (Syvanen (1999) Hum. Mutat. 13, 1-10). The technique of 'Targeting Induced Local Lesions in Genomes' (TILLING; McCallum et al. (2000) Nat. Biotechnol. 18, 455-457; Plant Physiol. 123, 439-442), which is a variant of (i) supra, can also be applied to rapidly identify an altered gene in e.g. chemically mutagenised plant individuals showing interesting phenotypes.

The use of these allelic variants in particular conventional breeding programmes, such as in marker-assisted breeding is also described; this may be in addition to their use in the methods according to the present invention. Such breeding programmes sometimes require the introduction of allelic variations in the plants by mutagenic treatment of a plant. One suitable mutagenic method is EMS mutagenesis. Identification of allelic variants then may take place by, for example, PCR. This is followed by a selection step for selection of superior allelic variants of the metallothionein sequence in question and which give rise to modified growth and development in a plant. Selection is typically carried out by monitoring growth performance of plants containing different allelic variants of the sequence in question, for example, different allelic variants of SEQ ID NO: 1. Monitoring growth performance can be done in a greenhouse or in the field. Further optional steps include crossing plants, in which the superior allelic variant was identified, with another plant. This could be used, for example, to make a combination of interesting phenotypic features.

Therefore, as mutations in the metallothionein gene may occur naturally, they may form the basis for selection of plants showing higher yield. Described herein is the use of a nucleic acid encoding a metallothionein or the use of a nucleic acid capable of modulating the activity and/or levels of a metallothionein in breeding programs.

Also described herein is the use of a nucleotide sequence capable of modulating expression of a nucleic acid encoding a metallothionein in breeding programmes. The nucleic acid sequence may be on a chromosome, or a part thereof, comprising at least the nucleic acid sequence encoding the metallothionein and preferably also one or more related family members. In an example of such a breeding programme, a DNA marker is identified which may be genetically linked to a gene capable of modulating expression of a nucleic acid encoding a metallothionein in a plant, which gene may be a gene encoding the metallothionein itself or any other gene which may directly or indirectly influence expression of the gene encoding a metallothionein and/or activity of the metallothionein itself. This DNA marker may then used in breeding programs to select plants having altered growth characteristics.

The methods may also be practised by introducing into a plant at least a part of a (natural or artificial) chromosome (such as a Bacterial Artificial Chromosome (BAC)), which chromosome contains at least a gene/nucleic acid sequence encoding a metallothionein (such as SEQ ID NO: 1 or SEQ ID NO: 3), preferably together with one or more related gene family members. Therefore, there is provided a method for modifying the growth and development of plants by introducing into a plant at least a part of a chromosome comprising at least a gene/nucleic acid encoding a metallothionein.

There is also provided a method for increasing plant seed yield, comprising increasing expression in a plant of a nucleic acid sequence encoding a metallothionein and/or increasing activity and/or levels in a plant of a metallothionein, wherein said nucleic acid sequence and said proteins include variants chosen from:

(i) a nucleic acid as represented by SEQ ID NO: 1 or encoding a metallothionein protein as represented by SEQ ID NO: 2; <u>(ii)</u> a nucleic acid encoding a metallothionein comprising the consensus sequence MSCCGG (N/S) CGCG (T/S/A) (G/A/S) C (K/Q/S) C ;

(iii) a metallothionein protein as represented by SEQ ID NO: 2
(iv) homologues, derivatives and active fragments of a metallothionein protein comprising the consensus sequence MSCCGG (N/S) CGCG (T/S/A) (G/A/S) C (K/Q/S) C.

According to the present invention, enhanced or increased expression of a nucleic acid is envisaged. Methods for obtaining enhanced or increased expression of genes or gene products are well documented in the art and include, for example, overexpression driven by a (strong) promoter, the use of transcription enhancers or translation enhancers. The term overexpression as used herein means any form of expression that is additional to the original wild-type expression level. The nucleic acid to be introduced into the plant and/or to be overexpressed is oriented in sense direction with respect to the promoter to which it is operably linked. Preferably, the nucleic acid to be overexpressed encodes a metallothionein, further preferably the nucleic acid sequence encoding the metallothionein is isolated from a dicotyledonous plant, preferably of the family Brassicaceae, further preferably wherein the sequence is isolated from *Arabidopsis thaliana,* most preferably the nucleic acid sequence is as represented by SEQ ID NO: 1 or a portion thereof, or encodes an amino acid sequence as represented by SEQ ID NO: 2 or a homologue, derivative or active fragment thereof. Alternatively, the nucleic acid sequence encoding the metallothionein is as represented by SEQ ID NO: 3 or is a portion thereof, or encodes an amino acid sequence as represented by SEQ ID NO: 4 or encodes a homologue, derivative or active fragment thereof. It should be noted that the applicability of the invention is not limited to use of the nucleic acid represented by SEQ ID NO: 1 nor to the nucleic acid sequence encoding the amino acid sequence of SEQ ID NO: 2, but that other nucleic acid sequences encoding homologues, derivatives or active fragments of SEQ ID NO: 2, or portions of SEQ ID NO: 1, or sequences hybridising with SEQ ID NO: 1 may be used in the methods of the present invention.

Also described herein is decreased expression of a nucleic acid sequence. Modulating gene expression (whether by a direct or indirect approach) encompasses altered transcript levels of a gene. Altered transcript levels can be sufficient to induce certain phenotypic effects, for example via the mechanism of cosuppression. Here the overall effect of overexpression of a transgene is that there is less activity in the cell of the protein encoded by a native gene having homology to the introduced transgene. Other examples of decreasing expression are also well documented in the art and include, for example, downregulation of expression by anti-sense techniques, co-suppression techniques, RNAi techniques, small interference RNAs (siRNAs), microRNA (miRNA), the use of ribozymes, etc. Therefore described herein is a method for modulating growth characteristics of plants, including technologies that are based on the synthesis of antisense transcripts, complementary to the mRNA of a metallothionein gene, or based on RNA interference. Plants having modified growth characteristics may be obtained by expressing a nucleic acid sequence encoding a metallothionein in either sense or antisense orientation. Techniques for downregulation are well known in the art. The terms "gene silencing" or "downregulation" of expression, as used herein, refer to lowering levels of gene expression and/or levels of active gene product and/or levels of gene product activity. Such decreases in expression may be accomplished by, for example, the addition of coding sequences or parts thereof in a sense orientation (if it is desired to achieve co-suppression). The growth of a plant may be modified by introducing into a plant an additional copy (in full or in part) of a metallothionein gene already present in a host plant. The additional gene will silence the endogenous gene, giving rise to a phenomenon known as co-suppression.

Genetic constructs aimed at silencing gene expression may comprise the metallothionein nucleotide sequence, for example as represented by SEQ ID NO: 1 (or one or more portions thereof) in a sense and/or antisense orientation relative to the promoter sequence. The sense or antisense copies of at least part of the endogenous gene in the form of direct or inverted repeats may be utilised in the methods according to the invention. The growth characteristics of plants may also be modified by introducing into a plant at least part of an antisense version of the nucleotide sequence represented, for example, by SEQ ID NO: 1. It should be clear that part of the nucleic acid (a portion) could achieve the desired result. Homologous anti-sense genes are preferred to heterologous anti-sense genes, homologous genes being plant genes, preferably plant genes from the same plant species, and heterologous genes being genes from non-plant species.

Another method for downregulation of gene expression or gene silencing comprises use of ribozymes, for example as described in Atkins et al. 1994 (WO 94/00012), Lenee et al. 1995 (WO 95/03404), Lutziger et al. 2000 (WO 00/00619), Prinsen et al. 1997 (WO 97/3865) and Scott et al. 1997 (WO 97/38116).

Gene silencing may also be achieved by insertion mutagenesis (for example, T-DNA insertion or transposon insertion) or by gene silencing strategies as described by, among others, Angell and Baulcombe 1998 (WO 98/36083), Lowe et al. 1989 (WO 98/53083), Lederer et al. 1999 (WO 99/15682) or Wang et al. 1999 (WO 99/53050). Expression of an endogenous gene may also be reduced if the endogenous gene contains a mutation. Such a mutant gene may be isolated and introduced into the same or different plant species in order to obtain plants having modified growth characteristics.

Advantageously, performance of the method according to the present invention results in plants having increased seed yield.

Modified growth characteristics as described herein also include modified growth, modified yield/biomass, modified architecture and a modified cell division, each relative to corresponding wild type plants. Preferably, the modified growth characteristics are improved growth characteristics and include modified architecture, increased yield/biomass with the proviso that increased yield is not increased metal accumulation, modified stress response with the proviso that the stress is not abiotic stress, and faster growth, each relative to corresponding wild type plants.

Growth characteristics as described herein comprise any one or more selected from: increased yield, increased biomass, increased total above ground area, increased plant height, increased number of tillers, increased number of primary panicles, increased number of secondary panicles, increased total number of seeds, increased number of filled seeds, increased total seed yield per plant, increased harvest index, increased thousand kernel weight, increased Tmid, increased T45 or A90, increased A42, altered cycling time and/or an altered growth curve. Described herein are methods to alter one of the above mentioned growth characteristics, without causing a penalty on one of the other growth characteristics, for example increase of the above ground green tissue area while retaining the same number of filled seeds and the same seed yield.

The term "increased yield" encompasses an increase in biomass in one or more parts of a plant relative to the biomass of corresponding wild-type plants. The term also encompasses an increase in seed yield, which includes an increase in the biomass of the seed (seed weight) and/or an increase in the number of (filled) seeds and/or in the size of the seeds and/or an increase in seed volume, each relative to corresponding wild-type plants. For maize, the increase of seed yield may be reflected in an increase of rows (of seeds) per ear and/or an increased number of kernels per row. An increase in seed size and/or volume may also influence the composition of seeds. An increase in seed yield could be due to an increase in the number and/or size of flowers. An increase in yield might also increase the harvest index, which is expressed as a ratio of the total biomass over the yield of harvestable parts, such as seeds; or thousand kernel weight. Increased yield also encompasses the capacity for planting at higher density (number of plants per hectare or acre).

Also modified cell division may contribute to yield increase. The term "modified cell division" encompasses an increase or decrease in cell division or an abnormal cell division/cytokinesis, altered plane of division, altered cell polarity, altered cell differentiation. The term also comprises phenomena such as endomitosis, acytokinesis, polyploidy, polyteny and endoreduplication. It can be envisaged that plants having increased biomass and height exhibit a modified growth rate when compared to corresponding wild-type plants. The term "modified growth rate" as used herein encompasses, but is not limited to, a faster rate of growth in one or more parts of a plant (including green biomass and including seeds), at one or more stages in the life cycle of a plant. The term "modified growth" encompasses enhanced vigour, earlier flowering, modified cycling time. Plants with modified growth may show a modified growth curve and may have modified values for their Tmid or T90 (respectively the time needed to reach half of their maximal area or 90% of their area, each relative to corresponding wild-type plants).

According to the present invention, performance of the methods according to the present invention result in plants having increased seed yield. Preferably, the increased seed yield includes an increased total number of seeds and/or increased total weight of seeds, each relative to control plants. Therefore, according to the present invention, there is provided a method for increasing seed yield of plants, which method comprises introduction and over expression in a plant of a nucleic acid sequence encoding a metallothionein comprising the consensus sequence MSCCGG (N/S) CGCG (T/S/A) (G/A/S) C (K/Q/S) C, preferably wherein the metallothionein is encoded by a nucleic acid sequence represented by SEQ ID NO: 1 or a portion thereof or sequences capable of hybridising therewith or wherein the metallothionein is represented by SEQ ID NO: 2 or is a metallothionein polypeptide comprising the consensus sequence MSCCGG (N/S) CGCG (T/S/A) (G/A/S) C (K/Q/S) C. Alternatively, the metallothionein comprising the consensus sequence MSCCGG (N/S) CGCG (T/S/A) (G/A/S) C (K/Q/S) C may be encoded by a nucleic acid sequence represented by SEQ ID NO: 3, or by a portion thereof or by sequences capable of hybridising therewith, or wherein the metallothionein is represented by SEQ ID NO: 4, or a homologue, derivative or active fragment of any thereof.

"Modified architecture" may be due to change in cell division. The term "architecture" as used herein encompasses the appearance or morphology of a plant, including any one or more structural features or combination of structural features thereof. Such structural features include the shape, size, number, position, texture, arrangement, and pattern of any cell, tissue or organ or groups of cells, tissues or organs of a plant, including the root, leaf, shoot, stem or tiller, petiole, trichome, flower, inflorescence (for monocotyledonous and dicotyledonous plants), panicles, petal, stigma, style, stamen, pollen, ovule, seed, embryo, endosperm, seed coat, aleurone, fibre, cambium, wood, heartwood, parenchyma, aerenchyma, sieve element, phloem or vascular tissue, amongst others. Modified architecture therefore includes all aspects of modified growth of the plant.

Preferably, the modified architecture includes modified number of primary panicles, modified plant height and modified total area, each relative to control plants. Described herein is a method for modifying the architecture of plants, particularly the number of primary panicles, plant height and plant area, which method comprises modulating expression of a nucleic acid sequence encoding a metallothionein and/or modulating activity of the metallothionein itself in a plant, preferably wherein the metallothionein is encoded by a nucleic acid sequence represented by SEQ ID NO: 1 or a portion thereof or sequences capable of hybridising therewith or wherein the metallothionein is represented by SEQ ID NO: 2 or a homologue, derivative or active fragment thereof. Alternatively, the metallothionein may be encoded by a nucleic acid sequence represented by SEQ ID NO: 3, or by a portion thereof or by sequences capable of hybridising with the aforementioned sequences, or wherein the metallothionein is represented by SEQ ID NO: 4, or a homologue, derivative or active fragment thereof.

Also described herein are genetic constructs and vectors to facilitate introduction and/or expression of the nucleotide sequences useful in the methods according to the invention are provided. Therefore, described herein is a gene construct comprising:

(i) a nucleic acid sequence encoding a metallothionein;
(ii) a GOS2 promoter for driving expression of the nucleic acid sequence of (i); and optionally
(iii) a transcription termination sequence.

Constructs useful in the methods according to the present invention may be constructed using recombinant DNA technology well known to persons skilled in the art. The gene constructs may be inserted into vectors, which may be commercially available, suitable for transforming into plants and suitable for expression of the gene of interest in the transformed cells. The genetic construct can be an expression vector wherein the nucleic acid sequence is operably linked to one or more control sequences allowing expression in prokaryotic and/or eukaryotic host cells.

The genetic construct described herein is an expression vector designed to overexpress the nucleic acid sequence. The nucleic acid sequence capable of over expression of a nucleic acid encoding a metallothionein and/or activity of the metallothionein itself may be a nucleic acid sequence encoding a metallothionein or a homologue, derivative or active fragment thereof comprising the consensus sequence MSCCGG (N/S) CGCG (T/S/A) (G/A/S) C (K/Q/S) C, such as any of the nucleic acid sequences described hereinbefore. A preferred nucleic acid sequence is the sequence represented by SEQ ID NO: 1 or a portion thereof or sequences capable of hybridising therewith or a nucleic acid sequence encoding a sequence represented by SEQ ID NO: 2 or a homologue, derivative or active fragment thereof comprising the consensus sequence MSCCGG(N/S)CGCG(T/S/A)(G/A/S)C(K/Q/S)C. For overexpression, this nucleic acid is cloned in the sense orientation relative to the control sequence to which it is operably linked.

Plants are transformed with a vector comprising the sequence of interest (i.e., the nucleic acid sequence capable of over expression of a nucleic acid encoding a metallothionein), which sequence is operably linked to one or more control sequences (at least a promoter). The terms "regulatory element", "control sequence" and "promoter" are all used herein interchangeably and are to be taken in a broad context to refer to regulatory nucleic acid sequences capable of effecting expression of the sequences to which they are ligated. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative which confers, activates or enhances expression of a nucleic acid molecule in a cell, tissue or organ. The terms "control sequence", "regulatory sequence", "regulatory element" and "promoter" are used interchangeably herein. The term "operably linked" as used herein refers to a functional linkage between the promoter sequence and the gene of interest, such that the promoter sequence is able to initiate transcription of the gene of interest.

Advantageously, any type of promoter may be used to drive expression of the nucleic acid sequence depending on the desired outcome. Preferably, the nucleic acid sequence capable of over expression of a gene encoding a metallothionein is operably linked to a constitutive promoter. The term "constitutive" as defined herein refers to a promoter that is expressed predominantly in at least one tissue or organ and predominantly at any life stage of the plant. Preferably the promoter is expressed predominantly throughout the plant. Preferably, the constitutive promoter is the GOS2 promoter from rice, or a promoter of similar strength and/or a promoter with a similar expression pattern. Alternatively, tissue specific promoters may be used. For example, in cases where increased seed yield is envisaged, the use of seed preferred, flower preferred, meristem preferred promoters or promoters active in dividing cells can be contemplated. Promoter strength and/or expression pattern can be analysed for example by coupling the promoter to a reporter gene and assay the expression of the reporter gene in various tissues of the plant. One suitable reporter gene well known to a person skilled in the art is *beta-glucuronidase.*

Examples of alternative constitutive promoters are presented in Table 1, and these promoters or derivatives thereof are useful for the methods of the present invention.

**Table 1:** Exemplary constitutive promoters for use in the performance of the present invention

| Gene source | Reference |
|---|---|
| Actin | McElroy et al, Plant Cell, 2: 163-171, 1990 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992 |
| ubiquitin | Christensen et al, Plant Mol. Biol. 18: 675-689, 1992 |
| rice cyclophilin | Buchholz et al, Plant Mol Biol. 25(5): 837-43, 1994 |
| maize H3 histone | Lepetit et al, Mol. Gen. Genet. 231:276-285, 1992 |
| actin 2 | An et al, Plant J. 10(1); 107-121, 1996 |

Optionally, one or more terminator sequences may also be used in the construct introduced into a plant. The term "terminator" encompasses a control sequence which is a DNA sequence at the end of a transcriptional unit which signals 3' processing and polyadenylation of a primary transcript and termination of transcription. Additional regulatory elements may include transcriptional as well as translational enhancers. Those skilled in the art will be aware of terminator and enhancer sequences which may be suitable for use in performing the invention. Such sequences would be known or may readily be obtained by a person skilled in the art.

The genetic constructs of the invention may further include an origin of replication sequence which is required for maintenance and/or replication in a specific cell type. One example is when a genetic construct is required to be maintained in a bacterial ceii as an episomai genetic element (e.g. plasmid or cosmid molecule). Preferred origins of replication include, but are not limited to, the f1-ori and colE1.

The genetic construct may optionally comprise a selectable marker gene. As used herein, the term "selectable marker gene" includes any gene which confers a phenotype on a cell in which it is expressed to facilitate the identification and/or selection of cells which are transfected or transformed with a nucleic acid construct of the invention. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin), to herbicides (for example bar which provides resistance to Basta; aroA or gox providing resistance against glyphosate), or genes that provide a metabolic trait (such as manA that allows plants to use mannose as sole carbon source). Visual marker genes result in the formation of colour (for example β-glucuronidase, GUS), luminescence (such as luciferase) or fluorescence (Green Fluorescent Protein, GFP, and derivatives thereof).

Described herein is the genetic construct as mentioned above comprising a metallothionein gene in sense orientation coupled to a promoter that is preferably a constitutive promoter, such as for example the rice GOS2 promoter. Therefore, described herein is a vector construct comprising an expression cassette to SEQ ID NO 7, which cassette comprises the rice GOS2 promoter, the *Arabidopsis metallothionein* gene presented in SEQ ID NO 2 and the T-zein + T-rubisco deltaGA transcription terminator sequence.

Alternatively, described herein is a vector construct comprising an expression cassette essentially similar to SEQ ID NO 7. A sequence essentially similar to SEQ ID NO 7 encompasses a first nucleic acid sequence encoding a protein homologous to SEQ ID NO 2 or hybridising to SEQ ID NO 1, which first nucleic acid is operably linked to a rice GOS2 promoter or a promoter with a similar expression pattern and/or which first nucleic acid is linked to a transcription termination sequence. Therefore described herein is a gene construct essentially similar to SEQ ID NO 7, comprising an expression cassette in which is located a nucleic acid sequence encoding a metallothionein protein, chosen from the group comprising:

(i) a nucleic acid sequence represented by SEQ ID NO: 1 or the complement strand thereof;
(ii) a nucleic acid sequence encoding an amino acid sequence represented by SEQ ID NO: 2 or homologues, derivatives or active fragments thereof comprising the consensus sequence MSCCGG(N/S)CGCG(T/S/A)(G/A/S)C(K/Q/S)C;
(iii) a nucleic acid sequence according to (i) to (ii) above which is degenerate as a results of the genetic code;
(iv) nucleic acid sequence which is an allelic variant of the nucleic acid sequences according to (i) to (iii);
(v) nucleic acid sequence which is an alternative splice variant of the nucleic acid sequences according to (i) to (iv);

Also described herein are plants obtainable by the methods according to the present invention, which plants have modified growth and development and which plants have increased metallothionein activity and/or increased expression of a nucleic acid sequence encoding a metallothionein. Preferably, the plants are transgenic plants comprising an introduced nucleic acid sequence encoding a metallothionein and having increased yield and/or biomass, characterized in that the transgenic plant has been selected for over expression of a nucleic acid sequence encoding a metallothionein and/or modulated activity of a metallothionein. Further preferably, the transgenic plant has been selected for increased expression of a nucleic acid encoding metallothionein.

According to another embodiment of the present invention, there is provided a method for the production of transgenic plants having increased seed yield comprising introduction and expression in a plant of a nucleic acid molecule encoding a metallothionein comprising: the consensus sequence MSCCGG (N/S) CGCG (T/S/A) (G/A/S) C (K/Q/S) C

More specifically, the present invention provides a method for the production of transgenic plants having increased seed yield, which method comprises:

(i) introducing into a plant or plant cell a nucleic acid sequence encoding metallothionein comprising the consensus sequence MSCCGG (N/S) CGCG (T/S/A) (G/A/S) C (K/Q/S) C;

(ii) regenerating and/or growing a plant from a transgenic plant cell.

The protein itself and/or the nucleic acid itself may be introduced directly into a plant cell or into the plant itself (including introduction into a tissue, organ or any other part of the plant). According to a preferred feature of the present invention, the nucleic acid sequence is preferably introduced into a plant by transformation. The nucleic acid sequence is preferably as represented by SEQ ID NO: 1 or a portion thereof or sequences capable of hybridising therewith, or is a nucleic acid sequence encoding an amino acid sequence represented by SEQ ID NO: 2 or a homologue, derivative or active fragment thereof comprising the consensus sequence MSCCGG (N/S) CGCG (T/S/A) (G/A/S) C (K/Q/S) C. Alternatively, the nucleic acid sequence is as represented by SEQ ID NO: 3 or a portion thereof or sequences capable of hybridising with any of the aforementioned sequences. The amino acid sequence may alternatively be a sequence as represented by SEQ ID NO: 4 or by homologues, derivatives or active fragments thereof comprising the consensus sequence MSCCGG (N/S) CGCG (T/S/A) (G/A/S) C (K/Q/S) C.

The term "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated therefrom. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell can then be used to regenerate a transformed plant in a manner known to persons skilled in the art.

Transformation of a plant species is now a fairly routine technique. Advantageously, any of several transformation methods may be used to introduce the gene of interest into a suitable ancestor cell. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., 1882, Nature 296, 72-74; Negrutiu I. et al., June 1987, Plant Mol. Biol. 8, 363-373); electroporation of protoplasts (Shillito R.D. et al., 1985 Bio/Technol 3, 1099-1102); microinjection into plant material (Crossway A. et al., 1986, Mol. Gen Genet 202, 179-185); DNA or RNA-coated particle bombardment (Klein T.M. et al., 1987, Nature 327, 70) infection with (non-integrative) viruses and the like. Transgenic rice plants expressing an metallothionein encoding gene are preferably produced via *Agrobacterium*-mediated transformation using any of the well known methods for rice transformation, such as described in any of the following: published European patent application EP 1198985 A1, Aldemita and Hodges (Planta, 199, 612-617, 1996); Chan et al. (Plant Mol. Biol. 22 (3) 491-506, 1993), Hiei et al. (Plant J. 6 (2) 271-282, 1994), which disclosures are incorporated by reference herein as if fully set forth. In the case of corn transformation, the preferred method is as described in either Ishida et al. (Nat. Biotechnol. 1996 Jun; 14(6): 745-50) or Frame et al. (Plant Physiol. 2002 May; 129 (1): 13-22), which disclosures are incorporated by reference herein as if fully set forth.

Generally after transformation, plant cells or cell groupings are selected for the presence of one or more markers which are encoded by plant-expressible genes co-transferred with the gene of interest, following which the transformed material is regenerated into a whole plant.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

Described herein are transgenic plants comprising an introduced nucleic acid encoding metallothionein and having modified growth and development as defined above, characterised in the modified growth and development is the consequence of modulated expression of a nucleic acid encoding a metallothionein. The transgenic plants are furthermore selected modulated expression of a nucleic acid encoding a metallothionein. Preferably, the transgenic plants are selected for increased expression of a nucleic acid encoding metallothionein.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

Described herein are plant cells or plants produced by any of the methods described herein, and to all plant parts and propagules thereof. Also described is the progeny of a primary transformed or transfected cell, tissue, organ or whole plant that has been produced by any of the aforementioned methods, the only requirement being that progeny exhibit the same genotypic and/or phenotypic characteristic(s) as those produced in the parent by the methods described herein Host cells containing an isolated nucleic acid molecule encoding a protein capable of modulating a metallothionein, preferably wherein the protein is a metallothionein. Preferred host cells are plant cells. Described herein are host cells or transgenic plants having altered growth characteristics, characterized in that said host cell or transgenic plant has modulated expression of a nucleic acid sequence encoding a metallothionein and/or modulated activity and/or level of a metallothionein. Harvestable parts of a plant such as but not limited to seeds, leaves, fruits, flowers, stem cultures, rhizomes, tubers and bulbs are also described.

The term "plant" as used herein encompasses whole plants, ancestors and progeny of the plants and plant parts, including seeds, shoots, stems, roots (including tubers), and plant cells, tissues and organs. The term "plant" also therefore encompasses suspension cultures, embryos, meristematic regions, callus tissue, leaves, seeds, roots, shoots, gametophytes, sporophytes, pollen, and microspores. Plants that are particularly useful in the methods of the invention include algae, ferns and all plants which belong to the superfamily Viridiplantae, in particular monocotyledonous and dicotyledonous plants including a fodder or forage legume, ornamental plant, food crop, tree, or shrub selected from the list comprising *Acacia* spp., *Acer* spp., *Actinidia* spp., *Aesculus* spp., *Agathis australis, Albizia amara, Alsophila tricolor, Andropogon* spp., *Arachis* spp, *Areca catechu, Astelia fragrans, Astragalus cicer, Baikiaea plurijuga, Betula* spp., *Brassica* spp., *Bruguiera gymnorrhiza, Burkea africana, Butea frondosa, Cadaba farinosa, Calliandra* spp, *Camellia sinensis, Canna indica, Capsicum* spp., *Cassia* spp., *Centroema pubescens, Chaenomeles* spp., *Cinnamomum cassia, Coffea arabica, Colophospermum mopane, Coronillia varia, Cotoneaster serotina, Crataegus* spp., *Cucumis* spp., *Cupressus* spp., *Cyathea dealbata, Cydonia oblonga, Cryptomeria japonica, Cymbopogon* spp., *Cynthea dealbata, Cydonia oblonga, Dalbergia monetaria, Davallia divaricata, Desmodium* spp., *Dicksonia squarosa, Diheteropogon amplectens, Dioclea* spp, *Dolichos* spp., *Dorycnium rectum, Echinochloa pyramidalis, Ehrartia* spp., *Eleusine coracana, Eragrestis* spp., *Erythrina* spp., *Eucalyptus* spp., *Euclea schimperi, Eulalia villosa, Fagopyrum* spp., *Feijoa sellowiana, Fragaria* spp., *Flemingia* spp, *Freycinetia banksii, Geranium thunbergii, Ginkgo biloba, Glycine javanica, Gliricidia* spp, *Gossypium hirsutum, Grevillea* spp., *Guibourtia coleosperma, Hedysarum* spp., *Hemarthia altissima, Heteropogon contortus, Hordeum vulgare, Hyparrhenia rufa, Hypericum erectum, Hyperthelia dissoluta, Indigo incarnata, Iris* spp., *Leptarrhena pyrolifolia, Lespediza* spp., *Lettuca* spp., *Leucaena leucocephala, Loudetia simplex, Lotonus bainesii, Lotus* spp., *Macrotyloma axillare, Malus* spp., *Manihot esculenta, Medicago sativa, Metasequoia glyptostroboides, Musa sapientum, Nicotianum* spp., *Onobrychis* spp., *Ornithopus* spp., *Oryza* spp., *Peltophorum africanum, Pennisetum* spp., *Persea gratissima, Petunia* spp., *Phaseolus* spp., *Phoenix canariensis, Phormium cookianum, Photinia* spp., *Picea glauca, Pinus* spp., *Pisum sativum, Podocarpus totara, Pogonarthria fleckii, Pogonarthria squarrosa, Populus* spp., *Prosopis cineraria, Pseudotsuga menziesii, Pterolobium stellatum, Pyrus communis, Quercus* spp., *Rhaphiolepsis umbellata, Rhopalostylis sapida, Rhus natalensis, Ribes grossularia, Ribes* spp., *Robinia pseudoacacia, Rosa* spp., *Rubus* spp., *Salix* spp., *Schyzachyrium sanguineum, Sciadopitys verticillata, Sequoia sempervirens, Sequoiadendron giganteum, Sorghum bicolor, Spinacia* spp., *Sporobolus fimbriatus, Stiburus alopecuroides, Stylosanthos humilis, Tadehagi* spp, *Taxodium distichum, Themeda triandra, Trifolium* spp., *Triticum* spp., *Tsuga heterophylla, Vaccinium* spp., *Vicia* spp., *Vitis vinifera, Watsonia pyramidata, Zantedeschia aethiopica, Zea mays,* amaranth, artichoke, asparagus, broccoli, Brussels sprouts, cabbage, canola, carrot, cauliflower, celery, collard greens, flax, kale, lentil, oilseed rape, okra, onion, potato, rice, soybean, straw, sugar beet, sugar cane, sunflower, tomato, squash tea, trees and algae amongst others. According to a preferred feature of the present invention, the plant is a crop plant comprising soybean, sunflower, canola, alfalfa, rapeseed or cotton. Further preferably, the plant according to the present invention is a monocotyledonous plant such as sugarcane, most preferably a cereal, such as rice, maize, wheat, millet, barley, oats, sorghum. However, it is envisaged that the methods of the present invention can be applied to a wide variety of plants, since the high degree of sequence conservation among the known eukaryotic metallothionein homologues suggests an equally conserved function in cellular metabolism.

The present invention also relates to use of a metallothionein protein comprising the consensus sequence MSCCGG (N/S) CGCG (T/S/A) (G/A/S) C (K/Q/S) C or to the use of a nucleic acid sequence encoding such a metallothionein in increasing seed yield in plants. The nucleic acid sequence is preferably as represented by SEQ ID NO: 1 or a portion thereof or sequences capable of hybridising therewith or is an amino acid sequence represented by SEQ ID NO: 2 or a homologue, derivative or active fragment thereof comprising the consensus sequence MSCCGG (N/S) CGCG (T/S/A) (G/A/S) C (K/Q/S) C. Preferably, the increase in seed yield comprises one or more of total number of seeds, total weight of seeds and a higher number of primary panicles.

Described herein is the use of a nucleic acid sequence encoding a metallothionein and homologues, derivatives and active fragments thereof and to use of the metallothionein itself and to homologues, derivatives and active fragments thereof as a growth regulator. The nucleic acid sequences hereinbefore described (and portions of the same and sequences capable of hybridising with the same) and the amino acid sequences hereinbefore described (and homologues, derivatives and active fragments of the same) are useful in modifying the growth characteristics of plants, as hereinbefore described. The sequences would therefore find use as growth regulators, to stimulate or inhibit plant growth. Therefore, described herein is a composition comprising a protein represented by SEQ ID NO 2 or a homologue, derivative or active fragment thereof for use in increasing yield and/or biomass of plants. Described herein is a composition comprising a nucleic acid as represented by SEQ ID NO 1 or a portion thereof or a sequence hybridising therewith for use in increasing yield and/or biomass of plants. Also described is a composition comprising a protein represented by any of the aforementioned amino acid sequences or homologues, derivatives or active fragments thereof for the use as a growth regulator.

Conversely, the sequences according to the present invention may also be interesting targets for agrochemical compounds, such as herbicides or growth stimulators. Accordingly, described herein is the use of the aforementioned nucleic acid sequences (or a portion of the same or sequences capable of hybridising with the same) or an amino acid sequence as hereinbefore described (or homologues, derivatives and active fragments of the same) as targets for an agrochemical compound, such as a herbicide or a growth stimulator.

The methods according to the present invention result in plants having increased seed yield as described hereinbefore. These advantageous growth characteristics may also be combined with other economically advantageous traits, such as further yield-enhancing traits, tolerance to various stresses, traits modifying various architectural features and/or biochemical and/or physiological features. The methods according to the present invention may also be practised by co-expression of a gene encoding a metallothionein protein in a plant with at least one other gene that cooperates with the gene encoding a metallothionein protein.

## Description of figures

The present invention will now be described with reference to the following figures in which:

**Figure 1:** Alignment of various plant metallothionein sequences (Cobbett and Goldsbrough, 2002). Conserved cysteines in the N-terminal and C-terminal regions are indicated with an asterisk. Abbreviations: At, *Arabidopsis vulgaris,* Bn, *Brassica napus,* Os, *Oryza sativa,* Ps *Pisum sativum,* Ms, *Medicago sativa,* Bo, *Brassica oleracea,* Ph, *Petunia hybrida, Sv, Silene vulgaris,* Ma, *Musa acuminata,* Ad, *Actinidia deliciosa,* Gh, *Gossipium hirsutum,* Pg, *Picea glauca,* Zm, *Zea mays,* Ta, *Triticum aestivum.*

**Figure 2:** Schematic presentation of the entry clone p33, containing CDS1585 within the AttL1 and AttL2 sites for Gateway® cloning in the pDONR201 backbone. CDS1585 is the internal code for the *Arabidopsis thaliana* metallothionein-like *AtMT2a* coding sequence. This vector contains also a bacterial kanamycine-resistance cassette and a bacterial origin of replication.

**Figure 3:** Binary vector for the expression in *Oryza sativa* of the *Arabidopsis thaliana* metallothionein-like *AtMT2a* gene (CDS1585) under the control of the rice GOS2 promoter (PRO0129). This vector contains a T-DNA derived from the Ti Plasmid, limited by a left border (LB repeat, LB Ti C58) and a right border (RB repeat, RB Ti C58)). From the left border to the right border, this T-DNA contains: a cassette for antibiotic selection of transformed plants; a cassette for visual screening of transformed plants; the PRO0129 - CDS1585 *-zein* and *rbcS-deltaGA* double terminator expression cassette (SEQ ID NO 7) for expression of the *Arabidopsis thaliana* metallothionein-like *AtMT2a* gene. This vector also contains an origin of replication from pBR322 for bacterial replication and a selectable marker (Spe/SmeR) for bacterial selection with spectinomycin and streptomycin.

**Figure 4:** Sequence listing

## Examples

The present invention will now be described with reference to the following examples, which are by way of illustration alone.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1984), Current Protocols in Molecular Biology, Current Protocols. Standard materials and methods for plant molecular work are described in Plant Molecular Biology Labfase (1993) by R.D.D. Croy, published by BIOS Scientific Publications Ltd (UK) and Blackwell Scientific Publications (UK).

### *Example 1. Cloning of CDS0851*

The *Arabidopsis* metallothionein coding sequence *AtMT2a* (CDS1585) was amplified by PCR using as template an *Arabidopsis thaliana* seedling cDNA library (Invitrogen, Paisley, UK). After reverse transcription of RNA extracted from seedlings, the cDNAs were cloned into pCMV Sport 6.0. Average insert size of the bank was 1.5 kb, and original number of clones was of $1.59 \times 10^7$ cfu. The original titer was determined to be $9.6 \times 10^5$ cfu/ml, and after a first amplification became $6 \times 10^{11}$ cfu/ml. After plasmid extraction, 200 ng of template was used in a 50 $\mu$l PCR mix. Primers prm03240 (SEQ ID NO 5) and prm03241 (SEQ ID NO 6), which include the AttB sites for Gateway recombination, were used for PCR amplification. PCR was performed using Hifi Taq DNA polymerase in standard conditions. A PCR fragment of 246 bp was amplified and purified also using standard methods. The first step of the Gateway procedure, the BP reaction, was then performed, during which the PCR fragment recombines in vivo with the pDONR201 plasmid to produce, according to the Gateway terminology, an "entry clone", p33 (Figure 1). Plasmid pDONR201 was purchased from Invitrogen, as part of the Gateway® technology.

### *Example 2. Vector construction for transformation with PRO0129-CDS1585 cassette*

The entry clone p33 was subsequently used in an LR reaction with p0640, a destination vector used for *Oryza sativa* transformation. This vector contains as functional elements within the T-DNA borders: a plant selectable marker; a screenable marker expression cassette; and a Gateway cassette intended for LR *in vivo* recombination with the sequence of interest already cloned in the entry clone. A rice GOS2 promoter for constitutive expression (PRO0129) is located upstream of this Gateway cassette.

After the LR recombination step, the resulting expression vector p34 (Figure 2) can be transformed into the *Agrobacterium* strain LBA4044 and subsequently to *Oryza sativa* plants.

### *Example 3. Transformation of rice with PRO0129 - CDS1585*

Mature dry seeds of *Oryza sativa* japonica cultivar Nipponbare were dehusked. Sterilization was done by incubating the seeds for one minute in 70% ethanol, followed by 30 minutes in 0.2% $HgCl_2$ and by 6 washes of 15 minutes with sterile distilled water. The sterile seeds were then germinated on a medium containing 2,4-D (callus induction medium). After a 4-week incubation in the dark, embryogenic, scutellum-derived calli were excised and propagated on the same medium. Two weeks later, the calli were multiplied or propagated by subculture on the same medium for another 2 weeks. 3 days before co-cultivation, embryogenic callus pieces were sub-cultured on fresh medium to boost cell division activity. The *Agrobacterium* strain LBA4044 harbouring the binary vector p3076 was used for co-cultivation. The *Agrobacterium* strain was cultured for 3 days at 28°C on AB medium with the appropriate antibiotics. The bacteria were then collected and suspended in liquid co-cultivation medium at an $OD_{600}$ of about 1. The suspension was transferred to a petri dish and the calli were immersed in the suspension during 15 minutes. Next, the callus tissues were blotted dry on a filter paper, transferred to solidified co-cultivation medium and incubated for 3 days in the dark at 25°C. Hereafter, co-cultivated callus was grown on 2,4-D-containing medium for 4 weeks in the dark at 28°C in the presence of a selective agent at a suitable concentration. During this period, rapidly growing resistant callus islands developed. Upon transfer of this material to a regeneration medium and incubation in the light, the embryogenic potential was released and shoots developed in the next four to five weeks. Shoots were excised from the callus and incubated for 2 to 3 weeks on an auxin-containing medium from which they were transferred to soil. Hardened shoots were grown under high humidity and short days in a greenhouse. Finally seeds were harvested three to five months after transplanting. The method yielded single locus transformants at a rate of over 50 % (Aldemita and Hodges, 1996, Chan *et al.,* 1993, Hiei *et al*., 1994).

### *Example 4. Evaluation of transgenic rice transformed with PRO0129-CDS1585*

Approximately 15 to 20 independent T0 transformants were generated. The primary transformants were transferred from tissue culture chambers to a greenhouse for growing and harvest of T1 seed. Seven events of which the T1 progeny segregated 3:1 for presence/absence of the transgene were retained. For each of these events, 10 T1 seedlings containing the transgene (hetero- and homo-zygotes), and 10 T1 seedlings lacking the transgene (nullizygotes), were selected by visual marker screening. The selected T1 plants were transferred to a greenhouse. Each plant received a unique barcode label to link unambiguously the phenotyping data to the corresponding plant. The selected T1 plants were grown on soil in 10 cm diameter pots under the following environmental settings: photoperiod= 11.5 h, daylight intensity= 30,000 lux or more, daytime temperature= 28°C or higher, night time temperature= 22°C, relative humidity= 60-70%. Transgenic plants and the corresponding nullizygotes were grown side-by-side at random positions. From the stage of sowing until the stage of maturity the plants were passed several times through a digital imaging cabinet. At each time point digital images (2048x1536 pixels, 16 million colours) were taken of each plant from at least 6 different angles.
The mature primary panicles were harvested, bagged, barcode-labelled and then dried for three days in the oven at 37°C. The panicles were then threshed and all the seeds collected. The filled husks were separated from the empty ones using an air-blowing device. After separation, both seed lots were then counted using a commercially available counting machine. The empty husks were discarded. The filled husks were weighed on an analytical balance and the cross-sectional area of the seeds was measured using digital imaging. This procedure resulted in the set of seed-related parameters described below.
These parameters were derived in an automated way from the digital images using image analysis software and were analysed statistically. A two factor ANOVA (analyses of variance) corrected for the unbalanced design was used as statistical model for the overall evaluation of plant phenotypic characteristics. An F-test was carried out on all the parameters measured of all the plants of all the events transformed with that gene. The F-test was carried out to check for an effect of the gene over all the transformation events and to verify for an overall effect of the gene, also named herein "global gene effect". If the value of the F test shows that the data are significant, than it is concluded that there is a "gene" effect, meaning that not only presence or the position of the gene is causing the effect. The threshold for significance for a true global gene effect is set at 5% probability level for the F test.
To check for an effect of the genes within an event, i.e., for a line-specific effect, a t-test was performed within each event using data sets from the transgenic plants and the corresponding null plants. "Null plants" or "Null segregants" or "Nullizygotes" are the plants treated in the same way as the transgenic plant, but from which the transgene has segregated. Null plants can also be described as the homozygous negative transformed plants. The threshold for significance for the t-test is set at 10% probability level. The results for some events can be above or below this threshold. This is based on the hypothesis that a gene might only have an effect in certain positions in the genome, and that the occurrence of this position-dependent effect is not uncommon. This kind of gene effect is also named herein a "line effect of the gene". The p-value is obtained by comparing the t-value to the t-distribution or alternatively, by comparing the F-value to the F-distribution. The p-value then gives the probability that the null hypothesis (i.e., that there is no effect of the transgene) is correct.
Vegetative growth and seed yield were measured according to the methods as described above. The inventors surprisingly found that the total number and total weight of seeds and the number of primary panicles were increased in the rice plants transformed with the metallothionein gene when compared the control plants without the *AtMt2a* gene.
The data obtained in the first experiment were confirmed in a second experiment with T2 plants. Three lines that had the correct expression pattern were selected for further analysis. Seed batches from the positive plants (both hetero- and homozygotes) in T1, were screened by monitoring marker expression. For each chosen event, the heterozygote seed batches were then retained for T2 evaluation. Within each seed batch an equal number of positive and negative plants were grown in the greenhouse for evaluation.
A total number of 120 *Mt2a* transformed plants were evaluated in the T2 generation, that is 40 plants per event of which 20 positives for the transgene, and 20 negatives.
Since two experiments with overlapping events had been carried out, a combined analysis was performed in addition to the above described analysis. This is useful to check consistency of the effects over the two experiments, and if this is the case, to accumulate evidence from both experiments in order to increase confidence in the conclusion. The method used was a mixed-model approach that took into account the multilevel structure of the data (i.e. experiment - event - segregants). P-values were obtained by comparing likelihood ratio test to chi square distributions.

### *Example 5. Results of the evaluation of transgenic plants transformed with PRO0129-CDS1585*

Upon analysis of the seeds as described above, the inventors found that plants transformed with the Mt2a gene construct had a higher number of primary panicles, a higher total number of seeds and a higher total weight of seeds compared to plants lacking the Mt2a transgene. Positive results obtained for plants in the T1 generation were again obtained in the T2 generation. Not only individual transgenic lines scored significantly better than the corresponding nullizygous control lines, but there was also a significant positive overall effect when all plants of all tested T2 events were evaluated, strongly indicating a global gene effect. An overview of the data is given in Table 2.

Table 2

| | T1 plants | | T2 plants | | Combined analysis |
|---|---|---|---|---|---|
| parameter | % difference | p-value | % difference | p-value | p-value |
| Total number of seeds | 14 | 0.0404 | 16 | 0.0184 | 0.0003 |
| Total weight of seeds | 9 | 0.2092 | 24 | 0.0091 | 0.0017 |
| Number of primary panicles | 16 | 0.0958 | 13 | 0.0907 | 0.0002 |

The % increase presents the average increase for all tested events. The p-values for the T1 and T2 plants stand for the p-value derived from the F-test and the p-values for the combined analysis were obtained by comparing likelihood ratio test to chi square distributions.

**Total number of seeds**

Total seed number per plant was measured by counting the number of husks harvested from a plant. Transgenic T1 lines showed an overall increase in total seed number of 14%, which increase was significant. This increase was confirmed in experiments with T2 plants, where a significant increase of 16% was measured. The combined analysis of T1 and T2 plants showed a global gene effect (p-value of 0.0003).

**Total seed yield**

The total seed yield (total weight of seeds) per plant was measured by weighing all filled husks harvested from a plant. On average, the increase in seed yield for T1 plants was 9%. These results were also observed in the T2 generation. The 3 tested lines had an average yield increase of 24%. This mean increase was statistically significant (p-value of 0.0091) and the combined analysis of the T1 and T2 plants showed there was a global gene effect (p-value of 0.0017).

**Number of primary panicles**

The tallest panicle and all the panicles that overlapped with the tallest panicle when aligned vertically were considered as primary panicles, and counted manually. There was an overall effect of the transgene on the number of panicles: the increase for the T1 plants was 16% and 13% for the T2 plants. These increases were significantly as evidenced by the p-values (respectively 0.0958 and 0.0907). The combined analysis demonstrated a global gene effect (p-value 0.0002).

A variation between the different transformation events (different plant events each transformed with the AtMT2a gene) was observed. It is well known to persons skilled in the art that the expression of transgenes in plants, and hence also the phenotypic effect due to expression of such transgene, can differ among different independently obtained transgenic lines and progeny thereof. The transgenes present in different independently obtained transgenic plants differ from each other by the chromosomal insertion locus as well as by the number of transgene copies inserted in that locus and the configuration of those transgene copies in that locus. Differences in expression levels can be ascribed to influence from the chromosomal context of the transgene (the so-called position effect) or from silencing mechanisms triggered by certain transgene configurations (e.g. inwards facing tandem insertions of transgenes are prone to silencing at the transcriptional or post-transcriptional level). Despite this variability, the analysis of the T1 and T2 plants and the combined analysis showed that the observed increases in yield are caused by a true global gene effect.

***Example 6: Modifying kernel yield in Zea mays:***

The invention described herein can also be used in maize. To this aim, the *Mt2a* gene, or the maize orthologue thereof is cloned under control of a suitable promoter like the rice GOS2 promoter or another constitutive promoter in a plant transformation vector suited for *Agrobacterium*-mediated corn transformation. Such vectors and methods for corn transformation have been described in literature (EP0604662, EP0672752, EP0971578, EP0955371, EP0558676, Ishida et al. 1996; Frame et al., 2002). Transgenic plants made by these methods are grown in the greenhouse for T1 seed production. Heritability is checked by progeny segregation analysis. Copy number of the transgene is checked by quantitative real-time PCR and/or Southern blot analysis. Expression levels of the transgene are determined by reverse PCR and/or Northern analysis. Transgenic lines with single copy insertions of the transgene and with varying levels of transgene expression are selected for T2 seed production through selfing or for crossing to different germplasm. Progeny seeds are germinated and grown in the field or in the greenhouse in conditions well adapted for maize (16:8 hr photoperiod, 26-28°C daytime and 22-24°C night time temperature) as well under water-deficient, nitrogen-deficient, and excess NaCl conditions. In the case of selfing, null segregants from the same parental line, as well as wild type plants of the same cultivar are used as controls. In the case of crossing, transgenics, null segregants and wild type plants of the same cultivar are crossed to a chosen parent and F1 plants from the transgenic cross are compared to F1 plants from the null segregant and the wild type crosses. The progeny plants resulting from the selfing or the crosses are evaluated on different biomass and growth parameters, including plant height, stem thickness, number of leaves, total above ground area, leaf greenness, time to maturity, flowering time, ear number, harvesting time. The seeds of these lines are also checked on various parameters, such as grain size, total grain yield per plant, and grain quality (starch content, protein content and oil content). Lines that are most significantly improved versus the controls for any of the above-mentioned parameters are selected for further field testing and marker-assisted breeding, with the objective of transferring the field-validated transgenic traits into commercial germplasm. Methods for testing maize for growth and yield-related parameters in the field are well established in the art, as are techniques for introgressing specific loci (such as transgene containing loci) from one germplasm into another.

## Claims

1. Method for increasing seed yield compared to corresponding wild type plants, comprising introducing and overexpressing in a plant an isolated nucleic acid encoding a metallothionein comprising the consensus sequence MSCCGG(N/S)CGCG(T/S/A)(G/A/S)C(K/Q/S)C.

2. Method according to claim 1, wherein said increased seed yield comprises increased total number of seeds and/or increased total weight of seeds, when compared to corresponding wild type plants.

3. Method according to claim 1 or 2, wherein expression of said nucleic acid encoding a metallothionein protein is driven by a constitutive promoter, preferably the rice GOS2 promoter.

4. Method for the production of a transgenic plant having increased seed yield according to claim 1, which method comprises:

(i) introducing into a plant or plant cell a nucleic acid sequence as specified in claim 1;
(ii) regenerating and/or growing a plant from a transgenic plant cell.

5. Use of a nucleic acid sequence encoding a metallothionein as specified in claim 1 in increasing seed yield in plants.

6. Use of a metallothionein protein as specified in claim 1 in increasing seed yield in plants.

7. Use according to claim 5 or 6, wherein said increased seed yield comprises increased total number of seeds and/or increased total weight of seeds, when compared to corresponding wild type plants.

## Patentansprüche

1. Verfahren zum Erhöhen des Samenertrags im Vergleich zu entsprechenden Wildtyppflanzen, bei dem man in einer Pflanze eine isolierte Nukleinsäure, die für ein Metallothionein, umfassend die Konsensussequenz MSCCG(N/S) CGCG(T/S/A)(G/A/S)C(K/Q/S)C, codiert, einführt und darin überexprimiert.

2. Verfahren nach Anspruch 1, wobei der erhöhte Samenertrag eine erhöhte Gesamtsamenzahl und/oder ein erhöhtes Samengesamtgewicht im Vergleich zu entsprechenden Wildtyppflanzen umfasst.

3. Verfahren nach Anspruch 1 oder 2, wobei die Expression der Nukleinsäure, die für ein Metallothioneinprotein codiert, von einem konstitutiven Promoter, vorzugsweise dem Reis-GOS2-Promoter, vorangetrieben wird.

4. Verfahren zur Herstellung einer transgenen Pflanze mit erhöhtem Samenertrag nach Anspruch 1, das Folgendes umfasst:

(i) Einführen einer Nukleinsäuresequenz gemäß Anspruch 1 in eine Pflanze oder Pflanzenzelle;
(ii) Regenerieren und/oder Heranziehen einer Pflanze aus einer transgenen Pflanzenzelle.

5. Verwendung einer Nukleinsäuresequenz, die für ein Metallothionein gemäß Anspruch 1 codiert, zum Erhöhen des Samenertrags bei Pflanzen.

6. Verwendung eines Metallothioneinproteins gemäß Anspruch 1 zum Erhöhen des Samenertrags bei Pflanzen.

7. Verwendung nach Anspruch 5 oder 6, wobei der erhöhte Samenertrag eine erhöhte Gesamtsamenzahl und/oder ein erhöhtes Samengesamtgewicht im Vergleich zu entsprechenden Wildtyppflanzen umfasst.

## Revendications

1. Procédé pour augmenter le rendement en graines comparé à des plantes de type sauvage correspondantes, comprenant l'introduction et la surexpression dans une plante d'un acide nucléique isolé codant pour une métallothionéine comprenant la séquence consensus MSCCGG(N/S)CGCG(T/S/A)(G/A/S)C(K/Q/S)C.

2. Procédé selon la revendication 1, dans lequel ledit rendement en graines augmenté comprend un nombre total de graines augmenté et/ou un poids total de graines augmenté, par rapport à des plantes de type sauvage correspondants.

3. Procédé selon la revendication 1 ou 2, dans lequel l'expression dudit acide nucléique codant pour une protéine métallothionéine est contrôlée par un promoteur constitutif, de préférence le promoteur GOS2 de riz.

4. Procédé pour la production d'une plante transgénique ayant un rendement en graines augmenté selon la revendication 1, ledit procédé comprend :

(i) l'introduction dans une plante ou une cellule de plante d'une séquence d'acide nucléique telle que spécifiée dans la revendication 1 ;
(ii) la régénération et/ou la culture d'une plante d'une cellule de plante transgénique.

5. Utilisation d'une séquence d'acide nucléique codant pour un métallothionéine telle que spécifiée dans la revendication 1 dans l'augmentation du rendement en graines dans des plantes.

6. Utilisation d'une protéine métallothionéine telle que spécifiée dans la revendication 1 dans l'augmentation du rendement en graines dans des plantes.

7. Utilisation selon la revendication 5 ou 6, dans laquelle ledit rendement en graines augmenté comprend un nombre total de graines augmenté et/ou un poids total de graines augmenté, par rapport à des plantes de type sauvage correspondantes.

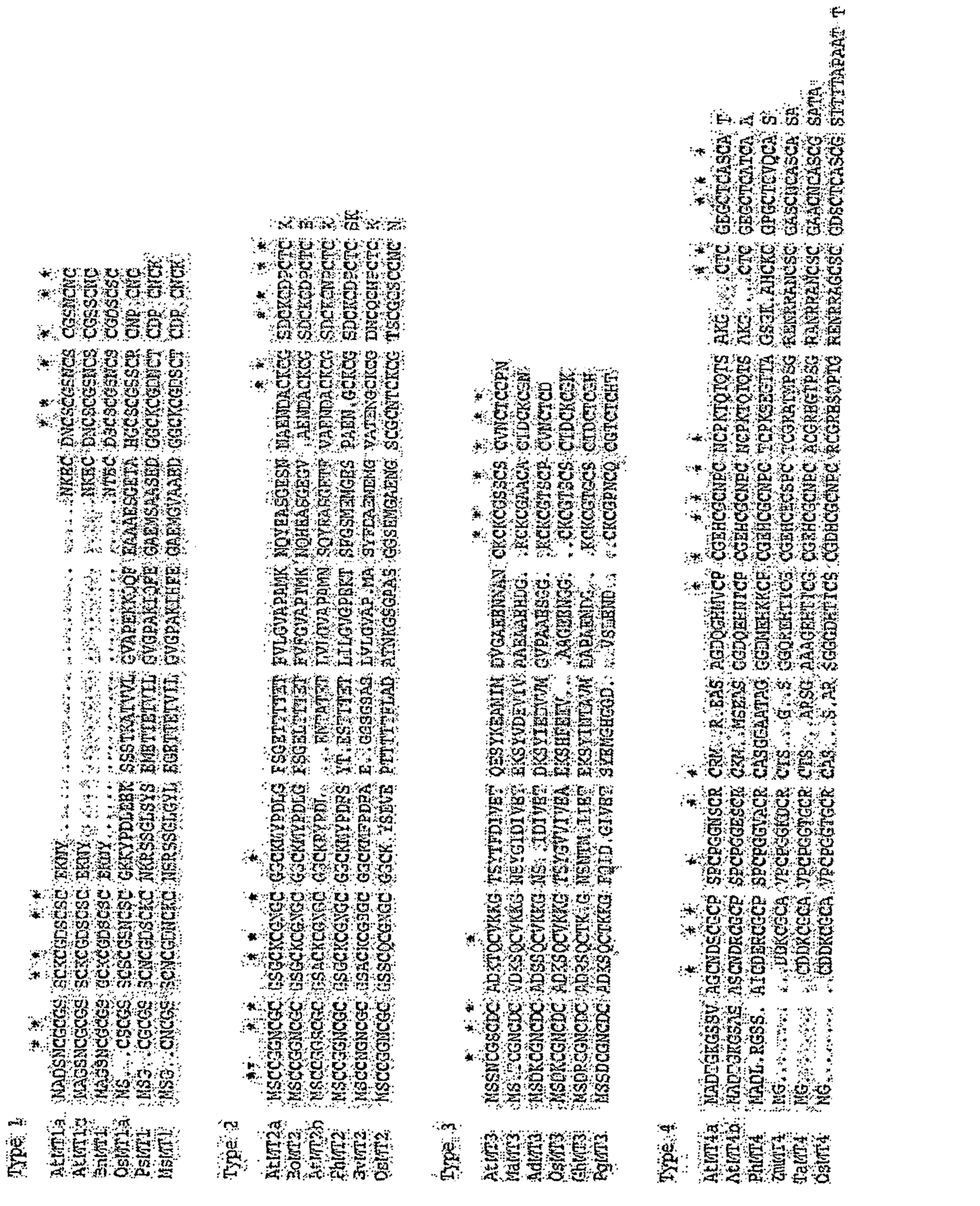

FIGURE 1

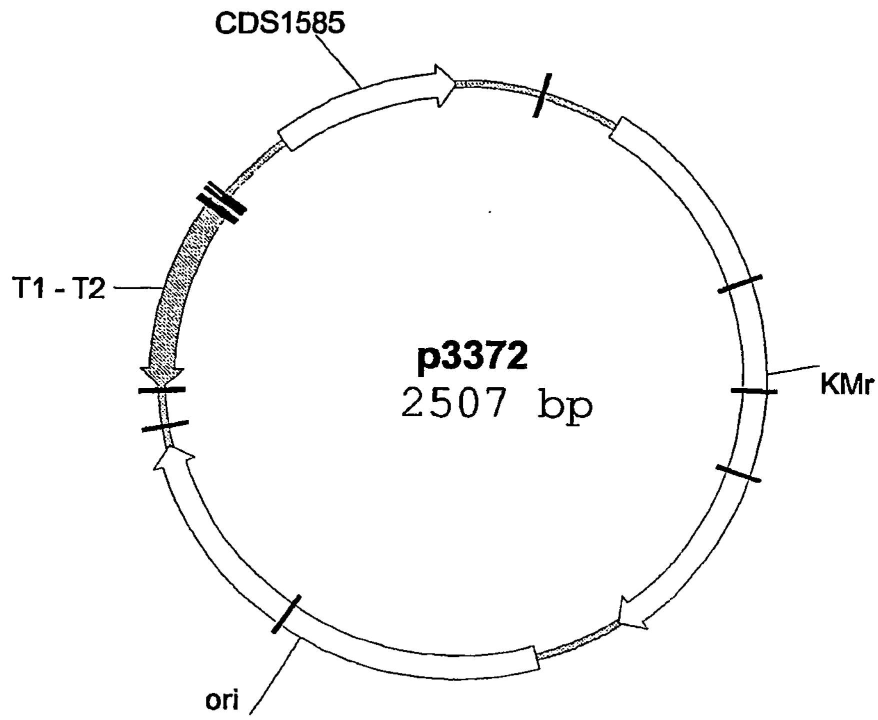


FIGURE 2

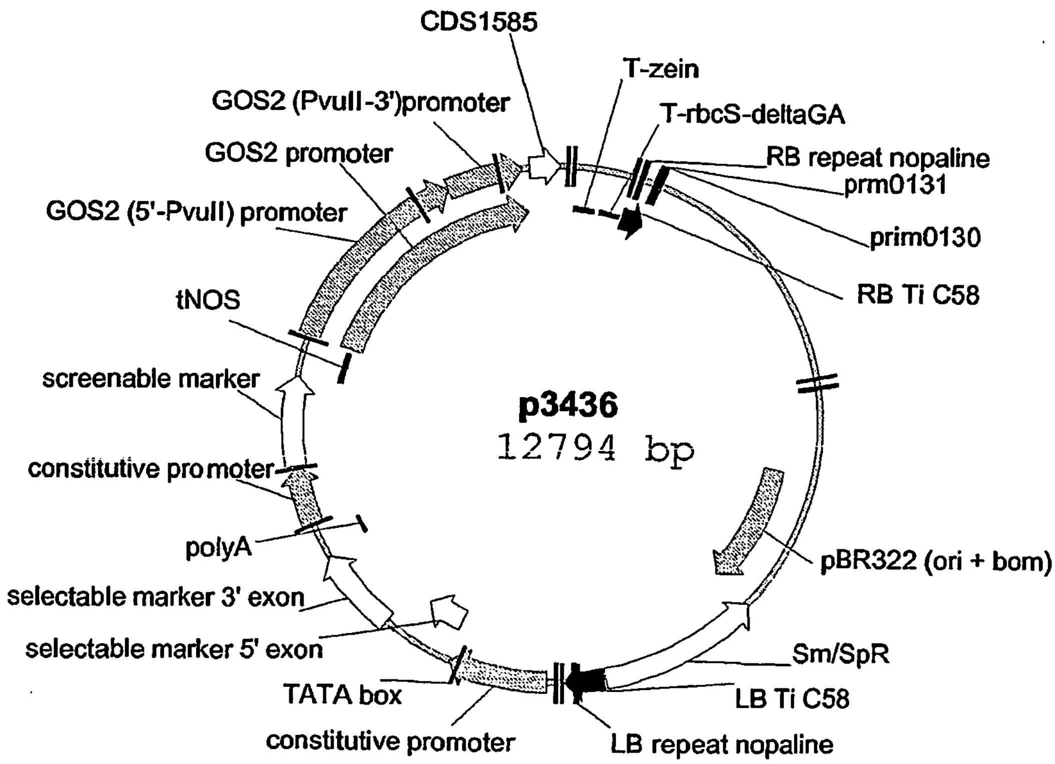


FIGURE 3

**SEQ ID NO 1: AtMT2a (CDS1585) coding sequence (start and stop in bold)**
ttttcattcataaattttcttcaatttgaattttctcgagaaaa**atg**tcttgctgtggagg
aaactgcggatgtggatctggctgcaagtgcggcaacggttgtggaggttgcaaaatgtacc
ctgacttgggattctccggcgagacaaccacaactgagacttttgtcttgggcgttgcaccg
gcgatgaagaatcagtacgaggcttcaggggagagtaacaacgctgagaacgatgcttgcaa
gtgtggatctgactgcaagtgtgatccttgcacctgcaag**tga**agaagccttttaaataag
cagagataatcgagtctctttaatta

**SEQ ID NO 2: AtMT2a (CDS1585) deduced protein sequence**
MSCCGGNCGCGSGCKCGNGCGGCKMYPDLGFSGETTTTETFVLGVAPAMKNQYEASGESNNA
ENDACKCGSDCKCDPCTCK

**SEQ ID NO 3: *A. thaliana* AtMT-1 cDNA, start and stop codon in bold.**
cattcataaattttcttcaatttgaattttctcgagaaaa**atg**tcttgctgtggaggaaac
tgcggatgtggatctggctgcaagtgcggcaacggttgtggaggttgcaaaatgtaccctga
cttgggattctccggcgagacaaccacaactgagacttttgtcttgggcgttgcaccggcga
tgaagaatcagtacgaggcttcaggggagagtaacaacgctgagagcgatgcttgcaagtgt
ggatctgactgcaagtgtgatccttgcacctgcaag**tga**agaagccttttaaataagcaga
gataatcgagtctctttaatntaattaagttattcaataagtaaaccatatataggatggtg
tttttaggtttggtttatgtgtaataatggcttcagcttatcttttagccgatcattgtctt
ttgtgtttgttttgatcatatcttttagatgtctagcaaatctgccatgtgatgagtttgta
cttccagtggaatgataataatattatagttttaaatcaaaaaaaaaaa

**SEQ ID NO 4: *A. thaliana* AtMT-1 deduced protein sequence**
MSCCGGNCGCGSGCKCGNGCGGCKMYPDLGFSGETTTTETFVLGVAPAMKNQYEASGESNNA
ESDACKCGSDCKCDPCTCK

**SEQ ID NO 5: prm03240**
GGGGACAAGTTTGTACAAAAAAGCAGGCTTCACAATGTCTTGCTGTGGAGGAA

**SEQ ID NO 6: prm03241**
GGGGACCACTTTGTACAAGAAAGCTGGGTTTCACTTGCAGGTGCAAG

**SEQ ID NO 7: Expression cassette for MT2a**
aatccgaaaagtttctgcaccgttttcacccccctaactaacaatatagggaacgtgtgctaa
atataaaatgagaccttatatatgtagcgctgataactagaactatgcaagaaaaactcatc
cacctactttagtggcaatcgggctaataaaaaagagtcgctacactagtttcgttttcct

## FIGURE 4

tagtaattaagtgggaaaatgaaatcattattgcttagaatatacgttcacatctctgtcat
gaagttaaattattcgaggtagccataattgtcatcaaactcttcttgaataaaaaatctt
tctagctgaactcaatgggtaaagagagagatttttttaaaaaaatagaatgaagatattc
tgaacgtattggcaaagatttaaacatataattatataatttatagtttgtgcattcgtca
tatcgcacatcattaaggacatgtcttactccatcccaattttatttagtaattaaagaca
attgacttattttattatttatctttttcgattagatgcaaggtacttacgcacacactt
tgtgctcatgtgcatgtgtgagtgcacctcctcaatacacgttcaactagcaacacatctct
aatatcactcgcctatttaatacatttaggtagcaatatctgaattcaagcactccaccatc
accagaccacttttaataatatctaaaatacaaaaaataatttacagaatagcatgaaaag
tatgaaacgaactatttaggttttcacatacaaaaaaaaaaagaattttgctcgtgcgcga
gcgccaatctcccatattgggcacacaggcaacaacagagtggctgcccacagaacaaccca
caaaaaacgatgatctaacggaggacagcaagtccgcaacaacctttaacagcaggctttg
cggccaggagagaggaggagaggcaaagaaaaccaagcatcctcctcctcccatctataaat
tcctccccccttttcccctctctatataggaggcatccaagccaagaagagggagagcacca
aggacacgcgactagcagaagccgagcgaccgccttcttcgatccatatcttccggtcgagt
tcttggtcgatctcttccctcctccacctcctcctcacagggtatgtgcccttcggttgttc
ttggatttattgttctaggttgtgtagtacgggcgttgatgttaggaaaggggatctgtatc
tgtgatgattcctgttcttggatttgggatagaggggttcttgatgttgcatgttatcggtt
cggtttgattagtagtatggttttcaatcgtctggagagctctatggaaatgaaatggttta
gggtacggaatcttgcgattttgtgagtaccttttgtttgaggtaaaatcagagcaccggtg
attttgcttggtgtaataaaagtacggttgtttggtcctcgattctggtagtgatgcttctc
gatttgacgaagctatcctttgtttattccctattgaacaaaaataatccaactttgaagac
ggtcccgttgatgagattgaatgattgattcttaagcctgtccaaaatttcgcagctggctt
gtttagatacagtagtccccatcacgaaattcatggaaacagttataatcctcaggaacagg
ggattccctgttcttccgatttgctttagtcccagaatttttttcccaaatatcttaaaaa
gtcactttctggttcagttcaatgaattgattgctacaaataatgcttttatagcgttatcc
tagctgtagttcagttaataggtaataccccctatagtttagtcaggagaagaacttatccga
tttctgatctccattttttaattatatgaaatgaactgtagcataagcagtattcatttggat
tatttttttattagctctcacccccttcattattctgagctgaaagtctggcatgaactgtc
ctcaattttgttttcaaattcacatcgattatctatgcattatcctcttgtatctacctgta
gaagtttcttttggttattccttgactgcttgattacagaaagaaatttatgaagctgtaa
tcgggatagttatactgcttgttcttatgattcatttcctttgtgcagttcttggtgtagct
tgccactttcaccagcaaagttcatttaaatcaactagggatatcacaagtttgtacaaaaa
agcaggcttcacaatgtcttgctgtggaggaaactgcggatgtggatctggctgcaagtgcg
gcaacggttgtggaggttgcaaaatgtaccctgacttgggattctccggcgagacaaccaca
actgagacttttgtcttgggcgttgcaccggcgatgaagaatcagtacgaggcttcaggga
gagtaacaacgctgagaacgatgcttgcaagtgtggatctgactgcaagtgtgatccttgca
cctgcaagtgaaacccagctttcttgtacaaagtggtgatatcacaagcccgggcggtcttc
tagggataacagggtaattatatccctctagatcacaagcccgggcggtcttctacgatgat
tgagtaataatgtgtcacgcatcaccatgggtggcagtgtcagtgtgagcaatgacctgaat
gaacaattgaaatgaaaagaaaaaaagtactccatctgttccaaattaaaattcattttaac
cttttaataggtttatacaataattgatatatgttttctgtatatgtctaatttgttatcat
ccgggcggtcttctagggataacagggtaattatatccctctagacaacacacaacaaataa
gagaaaaaacaaataatattaatttgagaatgaacaaaaggaccatatcattcattaactct
tctccatccatttccatttcacagttcgatagcgaaaaccgaataaaaaacacagtaaatta
caagcacaacaaatggtacaagaaaaacagttttcccaatgccataatactcgaac

**FIGURE 4 (continued)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9836084 A
- WO 9400012 A, Atkins
- WO 9503404 A, Lenee
- WO 0000619 A, Lutziger
- WO 973865 A, Prinsen
- WO 9738116 A, Scott
- WO 9836083 A, Angell and Baulcombe
- WO 9853083 A, Lowe
- WO 9915682 A, Lederer
- WO 9953050 A, Wang
- EP 1198985 A1
- EP 0604662 A
- EP 0672752 A
- EP 0971578 A
- EP 0955371 A
- EP 0558676 A
- EP 03076086 A


### Non-patent literature cited in the description


- **COBBETT ; GOLDSBROUGH.** *Annu Rev Plant Biol.,* 2002, vol. 53, 159-82
- **SUH et al.** *Mol Cells.,* 1998, vol. 8, 678-684
- Nutritional improvement of rice to reduce malnutrition in developing countries. **POTRYKUS I.** Plant Biotechnology 2002 and Beyond. 2002, 401-406
- **SCHEUHAMMER et al.** *Toxicol. Appl Pharmacol.,* 1986, vol. 82, 417-425
- **FABISIAK et al.** *Methods Enzymol.,* 2002, vol. 353, 268-281
- **NEEDLEMAN ; WUNSCH.** *J. Mol. Biol.,* 1970, vol. 48, 443-453
- **SMITH ; WATERMAN.** *Advances in Applied Mathematics,* 1981, vol. 2, 482-489
- **ALTSCHUL, S.F. ; GISH, W. ; MILLER, W. ; MYERS, E.W. ; LIPMAN, D.J.** *J. Mol. Biol.,* 1990, vol. 215, 403-410
- **W. R. PEARSON ; D. J. LIPMAN.** *Proc.Natl.Acad.Sci. USA,* 1988, vol. 85, 2444-2448
- **CREIGHTON.** Proteins. W.H. Freeman and Company, 1984
- **SAMBROOK et al.** Molecular Cloning: a laboratory manual. Cold Spring Harbor Laboratory Press, 2001
- Current Protocols in Molecular Biology. John Wiley & Sons, 1989
- **CHO et al.** *Nature Genet,* 1999, vol. 23, 203-207
- **LI-SUCHOLEIKI et al.** *Electrophoresis,* 1999, vol. 20, 1224-1232
- **FISCHER ; LERMAN.** *Proc. Natl. Acad. Sci. USA,* 1983, vol. 80, 1579-1583
- **ROSS et al.** *Biotechniques,* 2000, vol. 29, 620-629
- **TAPP et al.** *Biotechniques,* 2000, vol. 28, 732-738
- **KENNEY et al.** *Biotechniques,* 1998, vol. 25, 516-521
- **LANGEMEIER et al.** *Biotechniques,* 1994, vol. 17, 484-490
- **VIDAL-PUIG ; MOLLER.** *Biotechniques,* 1994, vol. 17, 490-496
- **SYVANEN.** *Hum. Mutat.,* 1999, vol. 13, 1-10
- **MCCALLUM et al.** *Nat. Biotechnol.,* 2000, vol. 18, 455-457
- *Plant Physiol.,* vol. 123, 439-442
- **MCELROY et al.** *Plant Cell,* 1990, vol. 2, 163-171
- **ODELL et al.** *Nature,* 1985, vol. 313, 810-812
- **NILSSON et al.** *Physiol. Plant,* 1997, vol. 100, 456-462
- **DE PATER et al.** *Plant J,* November 1992, vol. 2 (6), 837-44
- **CHRISTENSEN et al.** *Plant Mol. Biol.,* 1992, vol. 18, 675-689
- **BUCHHOLZ et al.** *Plant Mol Biol.,* 1994, vol. 25 (5), 837-43
- **LEPETIT et al.** *Mol. Gen. Genet,* 1992, vol. 231, 276-285
- **AN et al.** *Plant J.,* 1996, vol. 10 (1), 107-121
- **KRENS, F.A. et al.** *Nature,* June 1987, vol. 296, 72-74
- **NEGRUTIU I. et al.** *Plant Mol. Biol.,* vol. 8, 363-373
- **SHILLITO R.D. et al.** *Bio/Technol,* 1985, vol. 3, 1099-1102
- **CROSSWAY A. et al.** *Mol. Gen Genet,* 1986, vol. 202, 179-185
- **KLEIN T.M. et al.** *Nature,* 1987, vol. 327, 70

- **ALDEMITA ; HODGES.** *Planta,* 1996, vol. 199, 612-617
- **CHAN et al.** *Plant Mol. Biol,* 1993, vol. 22 (3), 491-506
- **HIEI et al.** *Plant J.,* 1994, vol. 6 (2), 271-282
- **ISHIDA et al.** *Nat. Biotechnol.,* June 1996, vol. 14 (6), 745-50
- **FRAME et al.** *Plant Physiol.,* May 2002, vol. 129 (1), 13-22
- **SAMBROOK.** Molecular Cloning: a laboratory manual. Cold Spring Harbor Laboratory Press, 2001
- Current Protocols in Molecular Biology. Current Protocols, 1984, vol. 1, 2
- **R.D.D. CROY.** Plant Molecular Biology Labfase. BIOS Scientific Publications Ltd (UK, 1993